(19) **Europäisches Patentamt / European Patent Office / Office européen des brevets**

(11) **EP 1 922 094 B1**

(12) **EUROPEAN PATENT SPECIFICATION**

(45) Date of publication and mention
of the grant of the patent:
**30.09.2015 Bulletin 2015/40**

(51) Int Cl.:
*A61L 27/54* (2006.01)     *A61L 27/40* (2006.01)
*A61L 27/44* (2006.01)     *A61L 27/56* (2006.01)
*A61L 27/58* (2006.01)     *A61P 35/00* (2006.01)
*A61K 31/704* (2006.01)    *A61K 31/337* (2006.01)
*A61M 31/00* (2006.01)     *A61L 27/26* (2006.01)
*A61L 27/28* (2006.01)

(21) Application number: **06775100.8**

(22) Date of filing: **14.08.2006**

(86) International application number:
**PCT/CA2006/001321**

(87) International publication number:
**WO 2007/019678 (22.02.2007 Gazette 2007/08)**

(54) **DEVICES FOR LYMPHATIC TARGETING**

VORRICHTUNGEN FÜR LYMPHATISCHES TARGETING

DISPOSITIFS PERMETTANT LE CIBLAGE LYMPHATIQUE

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **12.08.2005 US 707534 P**

(43) Date of publication of application:
**21.05.2008 Bulletin 2008/21**

(73) Proprietors:
• **Liu, Jiang**
**WuXi City, JiangSu Province 214125 (CN)**
• **Johnston, Michael Richard**
**Ketch Harbour NS B3V 3X2 (CA)**
• **Wu, Xiao Yu**
**Toronto, ON M2N 3X2 (CA)**

(72) Inventors:
• **Liu, Jiang**
**WuXi City, JiangSu Province 214125 (CN)**
• **Johnston, Michael Richard**
**Ketch Harbour NS B3V 3X2 (CA)**
• **Wu, Xiao Yu**
**Toronto, ON M2N 3X2 (CA)**

(74) Representative: **Brady, Paul Andrew et al**
**Abel & Imray
20 Red Lion Street
London WC1R 4PQ (GB)**

(56) References cited:
CA-A1- 2 457 564    CA-A1- 2 457 564
DE-A1- 4 201 461    US-A1- 2004 234 597
US-B1- 6 488 952

• HU Y ET AL: "CONTROLLED RELEASE FROM COATED POLYMER MICROPARTICLES EMBEDDED IN TISSUE-ENGINEERED SCAFFOLDS", JOURNAL OF DRUG TARGETING, HARWOOD ACADEMIC PUBLISHERS GMBH, DE, 1 January 2001 (2001-01-01), pages 431-438, XP008076932, ISSN: 1061-186X
• HAWLEY A.E. ET AL.: 'Lymph node localisation of biodegradable nanospheres surface modified with poloxamer and poloxamine block co-polymers' FEBS LETT. vol. 400, no. 3, 06 January 1997, pages 319 - 323, XP002394013
• NISHIOKA Y. ET AL.: 'Lymphatic targeting with nanoparticulate system' ADVANCED DRUG DELIVERY REVIEWS vol. 47, 2001, pages 55 - 64, XP002452038
• ILLUM L. ET AL.: 'Development of systems for targeting the regional lymph nodes for diagnostic imaging: in vivo behavior of colloidal PEG-coated magnetite nanospheres in the rat following inerstitial administration' PHARM. RES. vol. 18, no. 5, May 2001, pages 640 - 645, XP003008947

EP 1 922 094 B1

**(Cont. next page)**

• ROYCE S.M. ET AL.: 'Incorporation of polymer microspheres within fibrin scaffolds for the controlled delivery of FGF-1' J. BIOMATER. SCI. POLYMER EDN. vol. 15, no. 10, 2004, pages 1327 - 1336, XP008076933

• HU Y. ET AL.: 'Controlled release from coated polymer microparticles embedded in tissue-engineered scaffolds' JOURNAL OF DRUG TARGETING vol. 9, 2001, pages 431 - 438, XP008076932

**Description**

## FIELD OF THE INVENTION

The present invention relates to a novel targeted drug delivery system capable of delivering and retaining therapeutic agents in the lymphatic system, an anatomical location which is frequently affected by cancer and other diseases. In particular, this invention relates to the targeted delivery of therapeutic agents formulated in conjunction with micro- and/or nanoparticulate carriers to the lymphatics and lymph nodes and implantable devices containing the particulate carriers.

## BACKGROUND OF THE INVENTION

The lymphatic system is made up of an extensive network of thin walled vessels and lymph nodes that permeate almost every anatomical site in the body. The principal role of the lymphatic system is to carry plasma proteins, particulate matter and cells from interstitial fluid back to the blood stream. In addition the lymphatic system actively removes cell debris, microorganisms, and tumor cells. Many diseases affect the lymphatic system, some of which might be controlled if pharmaceuticals could be delivered into the lymphatic system more effectively.

Tumor cells can enter the lymphatic system and be carried to lymph nodes where secondary tumors (metastases) can grow. Since the interconnection between the lymphatic and venous systems is extensive, tumor within the lymphatic system often spreads through the blood to other organs.

In most cancers the initial sites of metastatic disease are the regional lymph nodes, which is a universal sign of tumor progression. Lymph node metastasis is regarded as one of the most important prognostic factors in accessing treatment options in patients with cancer. Once tumor involves the lymphatic system, the lymph nodes can act as holding reservoirs where the cancer cells can take root and seed into other regions of the body[i]. Even patients who have undergone potentially curative surgery still have a significant incidence of recurrence and subsequent death, which in part can be attributed to micrometastasis in the lymphatic system.

About twenty percent of Americans die from cancer, half of which are from lung, colorectal and breast cancer. Lung cancer is the leading cause of cancer deaths in both men and women. Despite recent advances in the treatment of lung cancer, the 5-year survival rate is still less than 15%. Its staging, prognosis and treatment are all intimately related to the degree of involvement of the lymphatic system. Once lymphatic metastasis has occurred, the chance of cure drops significantly. In non-small cell lung cancer (NSCLC) survival following surgery falls by about half if tumor is found in lymph nodes within the lung, and by another half if lymph nodes just outside of the lung (mediastinum) are involved. Even patients with early disease who have undergone potentially curative surgery still have a significant incidence of recurrence and subsequent death". Approximately 30% of patients with early stage NSCLC develop recurrent disease[iii,iv]. This suggests that occult micrometastatic tumor cells, which are not detectable by current staging techniques and conventional histopathologic methods, have already spread to the regional lymph nodes or distant organs at the time of surgery.

Colorectal cancer also causes significant morbidity and mortality. Accurate assessment of the lymph node metastasis is both prognostically and therapeutically important.[v] Surgery remains the mainstay of treatment for localized colorectal cancer. But without additional treatment nearly 50% of surgically treated patients die from relapse and metastatic progression. Systemic adjuvant chemotherapy with fluorouracil-based regimen only yields modest improvement of 5-year survival with Stage III disease.

Similarly, lymph node metastasis is one of the most important factors in evaluating the prognosis of breast cancer patients and correlates with disease-free and overall survival better than any other prognostic factor[vi]. As many as one-third of women with invasive breast cancer will have lymph node involvement at diagnosis. Five year survival rates drop from 80 percent in patients with no lymph node metastases to 45 to 50 percent in those patients who do have lymph node metastases.

Lymphoma is a primary tumor of the lymphatic system that may involve lymph nodes at sites throughout the body. Many lymphomas are effectively treated with systemic chemotherapy. However, some of the more virulent forms become resistant to standard chemotherapy and relapse despite initial response.

Control of lymphatic metastasis improves the outcome of many cancers. Presently, local-regional therapies, such as surgery and radiation, are the most effective means of treating regional lymphatics, but often do not completely eradicate all lymphatic metastatic disease. Systemic chemotherapy is limited by systemic side effects and often cannot effectively penetrate the lymphatic system, presumably because of a 'blood-lymph barrier'. Lymphatic drug delivery becomes even more compromised after extensive cancer surgery due to the disruption of blood and lymphatic vessels. Currently, there is a lack of effective treatment options for specifically targeting lymphatic metastasis. Therefore, effective therapeutic modalities based on a better understanding of the pathophysiology of lymphatic system are clearly needed to improve the treatment of tumor within the lymphatic system.

The distinct physiological function of the lymphatic system in the clearance of foreign particulate matters has generated interest in the use of microparticulate systems for the targeting of therapeutic agents to regional lymph nodes. Colloidal particles have an important role in characterizing the properties of the lymphatic system as well as a possible role in delivering drugs. A consistent finding is that the colloidal particles administered interstitially are mainly taken up by the lymphatic system and accumulate to varying degrees in the regional lymph nodes. The use of colloidal particles as radiodiagnostic agents has been reviewed by Strand et al.[vii] A wide range of materials were examined including solid particles, emulsions and vesicles (liposomes). Their distribution depends strongly on particle size, with colloids suggested for lymphoscintigraphy to have a median size of about 40-60 nm. Uptake into regional lymph nodes after, for example, subcutaneous administration is quite small and values from 1-10% are typical after 2-5 hours[viii]. This unique selective biodistribution led to the development of colloidal materials, such as liposomes[ix,x,xi], activated carbon particles[xii,xiii], emulsions[xiv,xv], lipids[xvi] and polymeric particulates[xvii,xvii] as drug carriers.

The peritoneum and pleura are the thin linings covering the inner surface of the peritoneal and pleural cavities and the surface of many organs and tissues they contain. Both peritoneum and pleura are rich in lymphatics. In the pleural cavity, there are many connections between lung and pleural lymph drainage, especially in the mediastinum. Similarly, extensive interconnections are found between peritoneal and visceral lymphatic drainage. Drainage tends to be towards those same regional lymph nodes where lymphatic metastases frequent occur. These connections provide a strong physiologic rationale for developing a lymphatic targeting strategy.

The parietal pleura is rich in lymphatic capillaries. Many stomata open directly to the pleural space, allowing particulate matter easy access into lymphatics. Shinohara H.[xix] examined pleural topography in the golden hamster using scanning electron microscopy. About 1,000 lymphatic stomata per pleural space were identified. The parietal pleural lymphatics drained lymph towards regional thoracic lymph nodes through multiple pathways. By eliminating the contribution of visceral pleural lymphatics in a pneumonectomy study, the present inventors showed that the dominant uptake of particles is through the parietal pleura[xx]. The exact mechanism of how the particles travel within the lymphatics is unclear. One explanation is that the particles are phagocytosed and carried by mononuclear cells, especially macrophages, to regional lymph nodes.

Delivering anticancer agents to regional lymph nodes has been attempted in the treatment of ovarian cancer, esophageal cancer, and breast cancer. In these studies, carbon or silica particles were used as drug carriers and injected subcutaneously (s.c.) or intratumourally. Both experimental and early clinical trials revealed considerable drug accumulation in lymph nodes and reduced cytotoxic drug levels in the plasma[xxi]. It is generally accepted that lymphatic uptake of intravenously (i.v.) administered colloidal particulate is unlikely since colloids cannot undergo transcapillary passage because of their size. After i.v. administration, they are mainly taken up by the macrophages of the liver and the spleen. As a result, it is difficult to modify the fate of drug carriers substantially unless different routes of administration are chosen.

Targeting for drug delivery purposes to the lymph nodes has been attempted using liposomes, emulsions and various non-lipid particle systems. Liposomes, being versatile, non-toxic and biocompatible lipid vesicles, have received the greatest amount of attention as carriers of various drugs. With liposome systems the best-recorded level of uptake in the lymph nodes is from 1-2% at 48 hours. This can be increased to about 5% by the attachment of antibodies.

There is limited information on lymphatic targeting using polymeric drug delivery systems. Although polylactides (PLA), polyglycolides (PGA), and their copolymers (PLGA) [xxii] have been developed for local delivery of chemotherapeutic agents, their primary design was for the treatment of cancerous peritonitis rather than targeting lymphatic metastasis [xxii]. The lymphatic uptake of polymer microspheres was an incidental finding during experiments [xxi xxv]. A PLGA nanosphere drug delivery system was particularly developed for subcutaneous (s.c.) lymphatic targeting [xxvi]. The particle sizes were less than 100 nm. The PLGA particles were surface engineered with PLA:PEG (Poly ethylene glycol) to increase the particle drainage from the injection site to regional lymph nodes.

The newly developed polymer-lipid hybrid nanoparticle containing doxorubicin complex (PLN-Dox) has been shown to enhance *in vitro* cytotoxicity towards wild-type and MDR human breast tumor cell lines *in vitro*. The PLN-Dox showed much higher *in vitro* cytotoxicity against the P-gp overexpressing cell line[xxviixxviii].

Due to the unique anatomy and physiology of pleura, the biodistribution of particulates following intrapleural administration has not been adequately investigated. Incidental finding of talc powder translocation to regional lymph nodes were reported following talc slurry pleurodesis [xxix xxx], which is a common procedure in the management of pleural effusion. Both human and animal experiments reveal that talc deposits in the pleural cavity can migrate to the mediastinal lymph nodes presumably through the pleural lymphatics [xxxi]. Recently, targeting liposome to mediastinal lymph nodes has been attempted via intrapleural administration [xxxii]. Pharmacokinetics and mediastinal node uptake of $^{111}$In-avidin and [99]mTc-biotin-liposomes were determined using scintigraphic imaging. Biodistribution results of $^{111}$In-avidin at 44 h showed 3.3% uptake in mediastinal nodes by pleural injection.

Since lymphatic micrometastasis can result in local tumor recurrence and potential systemic tumor spread, it is imperative to develop a drug targeting strategy to eliminate lymphatic micrometastasis. For the strategy to be successful, the anticancer drugs should be delivered from the site of application to the site of action. Conventional systemic chemotherapy cannot effectively deliver anticancer drugs to lymph nodes without incurring considerable side effects.

US2004/0234597 describes a pH triggered, site-specific targeted controlled delivery system comprising nanospheres encapsulated in pH sensitive microspheres. Active agents may be incorporated in the nanospheres and/ or microspheres. As the microspheres swell or dissolve within a pH range typically found in tumor tissue, the active ingredients are released selectively at these sites.

## SUMMARY OF THE INVENTION

The present invention relates to implantable devices containing a bioactive complex for the targeted delivery and retention of therapeutic agents to the lymphatic system, as described in claims 1-41.

In another embodiment the invention provides a process of preparing an implantable device according to the invention, as described in claim 42.

Other features and advantages of the present invention will become apparent from the following detailed description. It should be understood, however, that the detailed description and the specific examples while indicating preferred embodiments of the invention are given by way of illustration only, since various changes and modifications within the scope of the invention will become apparent to those skilled in the art from this detailed description.

## BRIEF DESCRIPTION OF THE DRAWINGS.

Figure 1 shows the biodistribution of $^{111}$In-aminpolystyrene with different sizes at 24 h after intrapleural administration in rats. (n=4); * $p<0.01$ (student $t$ test).

Figure 2 shows the biodistribution of $^{111}$In-aminpolystyrene with particle size of ~2 $\mu$m in rats as a function of time after intrapleural administration; (n=4); * $p<0.01$ (student $t$ test).

Figure 3 shows the *in vitro* drug release profile of PLGA-PTX microspheres.

Accumulative drug release was measured daily from day 1 to 21 and was then extended to every 5 days till day 51. The *in vitro* release of paclitaxel loaded PLGA microspheres was measured in PBS in triplicate at temperature of 37°C. 15 mg of paclitaxel loaded microspheres were suspended in 10 ml of PBS containing 0.1% (v/v) Tween 80. The tubes were tumbled end-over-end at 30 rpm at 37°C in a thermostatically controlled oven and at given time intervals, centrifuged at 10,000 rpm for 10 minutes and the top 8 ml of the supernatant was saved for analysis. The precipitated microspheres pellets were resuspended in the replaced releasing medium. The amount of paclitaxel was determined by extraction of paclitaxel into 3 ml of DCM. After dryness, the sample was reconstituted in 2 ml of 50:50 acetonitrile in water (v/v) and analyzed by HPLC.

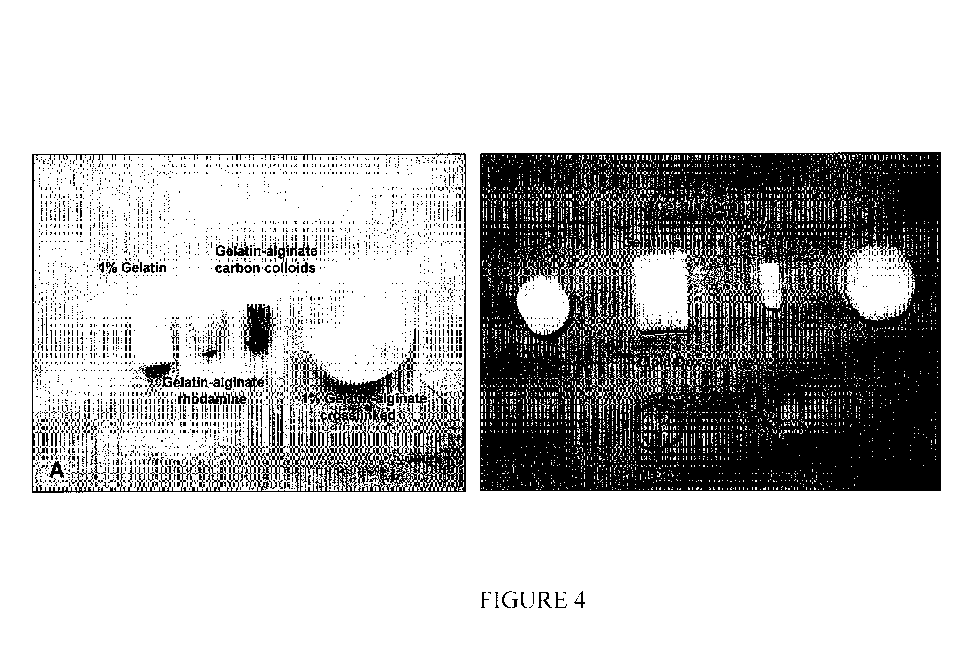

Figure 4 shows various gelatin and gelatin-alginate sponge samples with different sizes, geometries and contents.

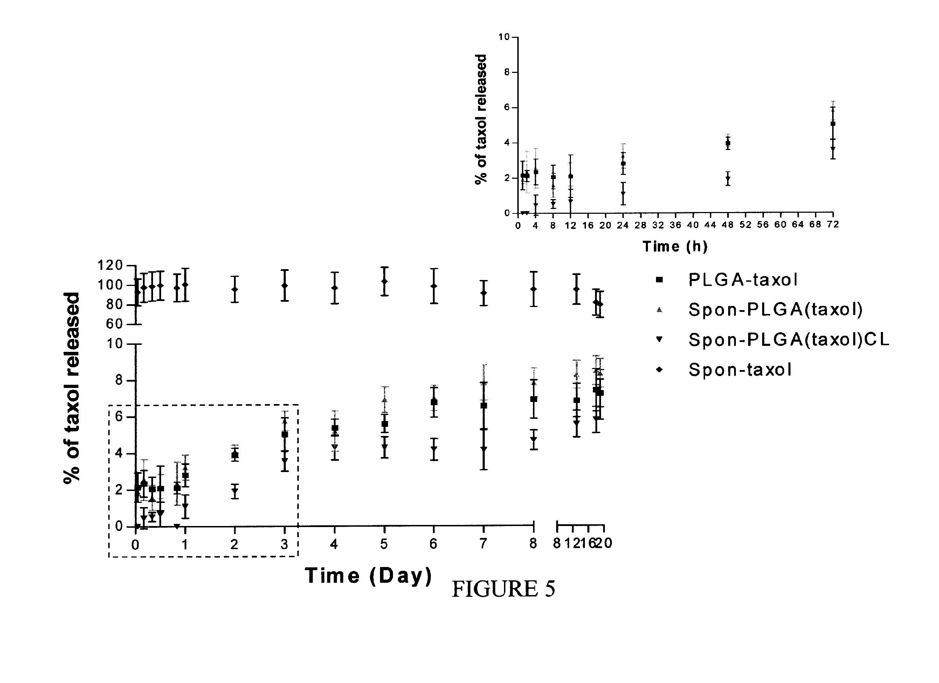

Figure 5 shows *in vitro* accumulative release profiles of paclitaxel from PLGA-PTX microspheres, gelatin sponges containing PLGA-PTX and PTX. Paclitaxel was measured in a phosphate buffer solutions containing calcium and magnesium and 0.5% (w/v) sodium dodecyl sulfate (SDS) at a concentration of 500 ng/ml for bacterial collagenase (type IV) in quadruplicate. The samples were incubated at 37°C and were shaken horizontally at 100 min[-1] in a shaking incubator. At given time intervals, the tubes were centrifuged at 5000 rpm for 15 min. 200 $\mu$l of supernatant was passed through a 0.22 $\mu$m syringe-driven filter with PVDF membrane. An aliquot of 100 $\mu$l filtrate was mixed with equal volume of acitonitrile. The mixture was centrifuged at 10000 rpm for 5min. The supernatant was aliquoted for HPLC detection. The amount of PTX was calculated from the calibration curves. Spon: Sponge; CL: Crosslinking.

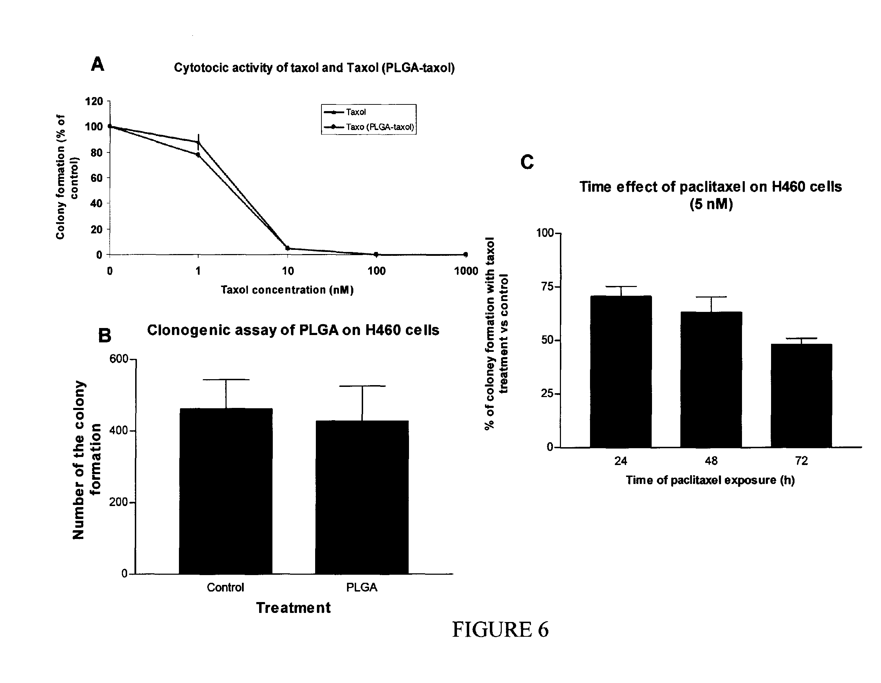

Figure 6 shows the results of an *in vitro* clonogenic assay of H460 lung cancer cells. The H460 cells were seeded at appropriate density in 6-cm cell-culture plates overnight before starting the treatment. H460 cells were treated with either free paclitaxel, PLGA placebo or PLGA-paclitaxel to assess the cytotoxicity. Paclitaxel, PLGA-paclitaxel compound equivalent and PLGA vehicle were solubilized in DMSO, and further diluted with RPMI-1640 medium (DMSO <0.1%). Sham treatment with this concentration of DMSO was implemented. A: *in vitro* cytotoxicity of free PTX comparing to PTX extracted from PLGA-PTX in the concentration range of 0.1 -1000 nM; B: comparison between PLGA treatment and sham control; C: cytotoxicity of paclitaxel as a function of exposure time at the concentration of $IC_{50}$ (5 nM).

## DETAILED DESCRIPTION OF THE INVENTION

The present inventors have developed a novel particulate drug delivery system capable of delivering bioactive agents such as antineoplastic agents to the lymphatic system, including regional lymphatics and lymph nodes and have developed an implantable device impregnated with a novel particulate drug delivery systems for targeted delivery to the lymphatic system. In a particular embodiment the lymphatic system is targeted through intrapleural, intraperitoneal or subcutaneous administration through surgical intervention.

Accordingly, the present invention includes an implantable device comprising a biocompatible and biodegradable matrix impregnated with a bioactive complex suitable for selectively targeting the lymphatic system, wherein the bioactive complex comprises one or more particle forming materials and one or more bioactive agents.

The "lymphatic system" as used herein refers to both the primary lymphatic system, including lymph capillaries, lymph vessels and lymph nodes and the secondary lymphoid tissues which are rich in lymphatics such as gut-associated lymphoid tissue, mesentery, Peyer's patches, omentum and accessory lymph tissue such as paranodal tissue at various body sites.

As used herein, the term "particles" refers to nanoparticles, microparticles, or both nanoparticles and microparticles.

The term "microparticles" is art-recognized, and includes microspheres and microcapsules, as well as structures that may not be readily placed into either of the above two categories, all with dimensions on average of less than 1000 microns. The term "microspheres" is art-recognized, and includes substantially spherical colloidal structures, e.g., formed from biocompatible polymers such as subject compositions, having a size ranging from about one or greater up to about 1000 microns. In general, "microcapsules", also an art-recognized term, may be distinguished from microspheres, because microcapsules are generally covered by a substance of some type, such as a polymeric formulation. If the structures are less than about one micron in diameter, then the corresponding art-recognized terms "nanosphere," "nanocapsule," and "nanoparticle" may be utilized. In certain embodiments, the nanospheres, nanocapsules and nanoparticles have an average diameter of about 500, 200, 100, 50 or 10 nm.

The term "bioactive complex" as used herein refers to a complex comprising one or more particle forming materials and one or more bioactive agents.

The term "particle forming material" as used herein refers to a material that is suitable for administration to a subject that can be combined with a bioactive agent and formed into a particle suitable for delivering the bioactive agent to the lymphatic system.

In one embodiment, the bioactive complex comprises microparticles or nanoparticles or a combination of micro- and nanoparticles of the particle forming material and the bioactive agent, said complex being of sufficient size to enter the lymphatic system. The term "sufficient size" as used herein would be readily understood by a person skilled in the art to mean of a size wherein the particles can migrate through the lymphatic vessels or be retained in the lymph nodes. In a particular embodiment, the particles range in size from about 50 nm to about 100 nm. In another embodiment the particles range in size from about 140 nm to about 1500 nm. In another embodiment the particles range in size from about 0.3 $\mu$m to about 11.2 $\mu$m. In another embodiment the particles range in size from about 0.7 $\mu$m to about 2 $\mu$m.

In a further embodiment the particle size may be varied to target different parts of the lymphatic system. For example, smaller size particles can be used to target the lymphatic vessels while larger size particles may be used to target the lymph nodes. In another embodiment of the invention the particle size may be varied to improve uptake by the lymphatic system, for example, vessels or lymph nodes located in different parts of the body. For example, for applications such as breast cancer and melanoma, particles under 100 nm are suitable while particles from about 1 $\mu$m to about 5 $\mu$m are suitable for intrapleural or intraperitoneal applications.

In embodiments of the invention the particles are formed from one or more pharmaceutically acceptable particle forming materials. Various particle forming materials can be used including pharmaceutically acceptable or biocompatible polymers, lipids, liposomes, metallic particles, magnetic particles, biotin, avidin and polysaccharides such as collagen, hyaluronic acid, albumin and gelatin, and derivatives thereof and combinations thereof.

The terms "pharmaceutically acceptable" or "biocompatible" are recognized in the art. In certain embodiments, the term includes compositions, polymers and other materials and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The term "polymer" refers to molecules formed from the chemical union of two or more repeating units, called monomers. Accordingly, included within the term "polymer" may be, for example, dimers, trimers and oligomers. The polymer may be synthetic, naturally-occurring or semisynthetic. In a suitable form, the term "polymer" refers to molecules which typically have a molecular weight (MW) greater than about 3000 and suitably greater than about 10,000 and a MW that is less than about 10 million, suitably less than about a million and more suitably less than about 200,000.

Examples of pharmaceutically acceptable or biocompatible polymers include, but are not limited to, polylactic acid, polyglycolic acid (PGA), polylactic-co-glycolic acid (PLGA), poly-lactic acid (PLA), polyvinyl pyrrolidones (PVP), polylactic acid-co-caprolactone, polyethylene glycol (PEG), polyethylene oxide (PEO), polystyrene, poly lactic acid-block-poly ethylene glycol, poly glycolic acid-block-poly ethylene glycol, poly lactide-co-glycolide-block-poly ethylene glycol, poly ethylene glycol-block-lipid, poly vinyl alcohol (PVA), polyester, poly(orthoester), poly(phosphazine), poly(phoasphate ester), polycaprolactaones, gelatin, collagen, a glycosaminoglycan, polyorthoesters, polysaccharides, polysaccharide derivatives, polyhyaluronic acid, polyalginic acid, chitin, chitosan, chitosan derivatives, cellulose, hydroxyethylcellulose, hydroxypropylcellulose, carboxymethylcellulose, polypeptides, polylysine, polyglutamic acid, albumin, polyanhydrides, polyhydroxy alkonoates, polyhydroxy valerate, polyhydroxy butyrate, proteins, polyphosphate esters, polyacrylamide (PAA), and derivatives and mixtures thereof.

The term "lipid", as used herein, refers to non-polymeric small organic, synthetic or naturally-occurring, compounds which are generally amphipathic and biocompatible. The lipids typically comprise a hydrophilic component and a hydrophobic component. Exemplary lipids include, for example, fatty acids, fatty acid esters, neutral fats, phospholipids, glycolipids, aliphatic alcohols, waxes, terpenes, steroids and surfactants. The term lipid is also meant to include derivatives of lipids. More specifically the term lipids includes but is not limited to phosphatidylcholine, phosphatidylethanolamine, phosphatidylserine, sphingomyelin as well as synthetic phospholipids such as dimyristoyl phosphatidylcholine, dipalmitoyl phosphatidylcholine, distearoyl phosphatidylcholine, distearoyl phosphatidylglycerol, dipalmitoyl phosphatidylglycerol, dimyristoyl phosphatidylserine, distearoyl phosphatidylserine and dipalmitoyl phosphatidylserine.

In an embodiment of the invention, the derivatives of the particle forming material are surface modified derivatives wherein the surface modification alters the hydrophilicity, hydrophobicity or otherwise alters the characteristics of the material so that it is suitable or targeted for specific areas in the body.

Suitable embodiments of the invention include particles made from degradable polymers such as polylactides (PLA), polyglycolides (PGA) and their copolymers (PLGA). One advantage of these degradable polymers is that they can be broken down into biologically acceptable molecules e.g. lactic or glycolic acid and water that are metabolized and removed from the body via normal metabolic pathways. For example, PLA/PLGA degrade by bulk hydrolysis in water. The rate of degradation of the polymer normally controls the release of encapsulated therapeutic agent. Therefore, depending on the therapy required for a particular drug, the release kinetics of that drug from its polymer matrix can be controlled by selecting a PLA or PLGA with appropriate physicochemical characteristics such as molecular weight and copolymer composition.

Particles may also be made from polystyrene particles that have been surface modified to alter their hydrophilicity, for example by grafting of hydrophilic polymers such as N-isopropylacrylamide (NIPAm) and/or methacrylic acid (MAA).

Since the anatomic and physiological features of the lymphatic system varies among different compartments of the body, potential exists to further improve targeting efficacy by modifying physicochemical properties of the polymer. One of the most important factors influencing the lymphatic particle distribution is particle size. The present inventors utilized $^{111}$Indium (In) labeled aminopolystyrene beads in three different sizes (~300 nm, ~2 $\mu$m, and ~11 $\mu$m) to investigate particle size effect on lymphatic particle distribution through intrapleural administration. The study revealed that ~2 $\mu$m particles have significantly higher lymphatic absorption compared to that of the two other sizes. The particles also exhibited reasonable dwelling time in the regional lymph nodes. Much smaller particle sizes (<100 nm) have been reported to be used for s.c. lymphatic delivery. For subcutaneous administration, the optimum size is between 10 nm and 50 nm. Recent studies have shown that particles larger than a few hundred nanometers in diameter are preferentially retained at the site of injection as they are too big to navigate through the interstitial space to join the lymph flow as it is required[xxxiii]. Only when particles are administered intraperitoneally or intrapleurally does size, within the nanometer range, become of less importance as drainage is simply from a cavity into the surface lymphatics, and hence no diffusion through the interstitial space is necessary [xxxiv]. The open junctions on the lymphatic wall are the only size limitation barriers to lymphatic uptake from the peritoneal cavity [xxxv]. The present inventors have shown that carbon colloids with size ranges from 700 to 1500 nm given intrapleurally provide better lymph node distribution than that of two other smaller sized particles. It appears that particles of approximately 2 $\mu$m are an appropriate size for intrapleural lymphatic targeting to regional lymph nodes. One possible explanation for the low uptake of small particles in lymph nodes is that, even though these particles may have easy access to pleural lymphatics, they fail to be retained in regional lymph nodes.

Despite the size effect on particle distribution in the lymphatic system, optimal size may vary between human and other mammals or in the animals with different species.

The particles of the invention are used to target bioactive agents to the lymphatic system including lymph nodes. Accordingly, in another embodiment, the particles of the invention contain a bioactive agent required to reach the lymphatics and/or lymph nodes.

The term "bioactive agent" as used herein refers to any therapeutic or diagnostic substance that is delivered to a bodily conduit of a living being to produce a desired, usually beneficial, effect.

In a suitable embodiment the bioactive agent is an antineoplastic agent. In one embodiment, the bioactive agent is an anti-proliferative or anti-metastatic drug. In suitable embodiments, the anti-proliferative or anti-metastatic drug includes, in general, microtubule-stabilizing agents (such as paclitaxel, docetaxel or their derivatives or analogs); alkylating agents; antimetabolites; epidophyllotoxin; an antineoplastic enzyme; a topoisomerase inhibitor; procarbazine; mitoxantrone; platinum coordination complexes; biological response modifiers and growth inhibitors; and haematopoietic growth factors. Exemplary classes of antineoplastic agents include, for example, the anthracycline family of drugs, the vinca drugs, the mitomycins, the bleomycins, the cytotoxic nucleosides, the taxanes, the epothilones, discodermolide, the pteridine family of drugs, diynenes and the podophyllotoxins. Members of those classes include, for example, doxorubicin, carminomycin, daunorubicin, aminopterin, methotrexate, methopterin, dichloro-methotrexate, mitomycin C, porfiromycin, trastuzumab (Herceptin.TM.), 5-fluorouracil, 6-mercaptopurine, gemcitabine, cytosine arabinoside, podo-

phyllotoxin or podo-phyllotoxin derivatives such as etoposide, etoposide phosphate or teniposide, melphalan, vinblastine, vincristine, leurosidine, vindesine, leurosine, paclitaxel and the like. Other useful antineoplastic agents include estramustine, cisplatin, carboplatin, cyclophosphamide, bleomycin, gemcitibine, ifosamide, melphalan, hexamethyl melamine, thiotepa, cytarabin, idatrexate, trimetrexate, dacarbazine, L-asparaginase, camptothecin, CPT-11, topotecan, pyridobenzoindole derivatives, interferons and interleukins.

Still other representative anti-proliferative or anti-metastatic drugs include: alkylating agents such as nitrogen mustards, for instance mechlorethamine, cyclophosphamide, melphatan and chlorambucil, alkyl sulpronates such as busulphan, nitrosoureas such as carmustine, lomusine, semustine and streptozocin, triazenes such as dacarbazine, antimetabolites such as folic acid analogues, for instance methotrexate, pyrimidine analogues such as fluorouracil and cytarabine, purine analogues such as mercaptopurine and thioguanine, natural products such as vinca alkaloids, for instance vinblastine, vincristine and vendesine, epipodophyllotoxins such as etoposide and teniposide, antibiotics such as dactinomycin, daunorubicin, doxorubicin, bleomycin, plicamycin and mitomycin, enzymes such as L-asparaginase, substituted ureas such as hydroxyurea, methylhydrazine derivatives such as procarbazine, adrenocorticoid suppressants such as mitotane and aminoglutethimide, hormones and antagonists such as adrenocorticosteroids such as prednisone, progestins such as hydroxyprogesterone caproate, methoxyprogesterone acetate and megestrol acetate, oestrogens such as diethylstilboestrol and ethinyloestradiol, antioestrogens such as tamoxifen, and androgens such as testosterone propionate and fluoxymesterone.

Further embodiments include moieties that suppress lymphangiogenesis such as vascular endothelial growth factor C, D (VEGF-C, D) antibody or the antibody of its receptor VEGFR-3, and moieties to suppress angiogenesis, such as anti epidermal growth factor receptor (EGFR) agent, various small molecules working on various anticancer signal pathways such as integrin linked kinase (ILK) inhibitor, matrix metalloproteinase (MMP) inhibitor, macromolecules, antioxidants, cytokines, chemokines, antisense, oligonucleotides, LyP-1 peptide-coated qdots to home to the lymphatics, hormones and hormone antagonists.

In suitable embodiments of the invention the bioactive agent is paclitaxel or a paclitaxel derivative or doxorubicin.

In another embodiment of the invention more than one bioactive agent can be incorporated into the micro particles. Multiple bioactive agents can be used in combination to provide synergistic effects or to provide multiple types of treatment. For example two or more complimentary therapeutic agents may be incorporated to provide complimentary forms of treatment, or tissue sensitizing agent may be combined with a therapeutic agent to provide a synergistic effect or a therapeutic agent and a radiation sensitizer may be combined in order to treat a malignancy with a combination of chemotherapy and radiation. Suitable combination of bioactive agents would be known to one of skill in the art.

In another embodiment of the invention, one or more bioactive agents that are independent of the bioactive complex may be incorporated into the biocompatible and biodegradable matrix. With the combination of a free bioactive agent and the bioactive complex comprising the bioactive agent, an optimal pharmokinetic profile for the bioactive agent may be achieved. The release of the free bioactive agent will provide an initial high agent concentration while the particles in the complex will slowly release the agent to maintain the agent level. This will allow the agent concentration to be maintained in the therapeutic window for a longer period of time. Accordingly the lethal dose ($LD_{50}$) and maximum tolerated dose (MTD) may be increased, enhancing the safety profile of the bioactive agent, while reducing the plasma effective concentration $EC_{50}$, and thereby enhancing the efficacy of the bioactive agent. In this embodiment, the free bioactive agent may also be different from the bioactive agent in the complex provide an alternative means for delivering synergistic or combination therapies at different release rates.

The incorporation of the bioactive agents into the particle forming material and preparation of nano- or microparticles may be done using any procedure known in the art. Various formulation methods can be used including spray drying, double emulsion, solvent evaporation and the like. For example in spray drying, the bioactive agent and the particle forming material are combined in a suitable solvent. The resulting solution or suspension is then spray-dried to form micro- or nanoparticles. The inlet temperature, outlet temperature, spray flow control, feed spray rate, aspirator level, and atomizing pressure can all be adjusted to produce particles having specific physical properties such as size and shape.

The present invention includes an implantable device for targeting bioactive agents to the lymphatic system or lymph nodes. The implantable device is a biocompatible, biodegradable matrix impregnated with particles containing a therapeutic agent.

Any suitable materials may be used to form the biocompatible matrix. In an embodiment of the invention, the material used to form the biocompatible matrix includes different types of polymers such as homopolymers or copolymers (including alternating, random, and block copolymers), they may be cyclic, linear or branched homopolymers or copolymers (including alternating, random and block copolymers), they may be cyclic, linear or branched (e.g., polymers have star, comb or dendritic architecture), they may be natural or synthetic, they may be thermoplastic or thermosetting, and they may be hydrophobic, hydrophilic or amphiphilic.

Polymers for use in the biocompatible matrix may be selected, for example, from the following: polycarboxylic acid polymers and copolymers including polyacrylic acids; acetal polymers and copolymers; acrylate and methacrylate polymers and copolymers (e.g., n-butyl methacrylate); cellulosic polymers and copolymers, including cellulose acetates, cellulose nitrates, cellulose propionates, cellulose acetate butyrates, cellophanes, rayons, rayon triacetates, and cellulose ethers such as carboxymethyl celluloses and hydoxyalkyl celluloses; polyoxymethylene polymers and copolymers; polyimide polymers and copolymers such as polyether block imides, polyamidimides, polyesterimides, and polyetherimides; polysulfone polymers and copolymers including polyarylsulfones and polyethersulfones; polyamide polymers and copolymers including nylon 6,6, nylon 12, polycaprolactams and polyacrylamides; resins including alkyd resins, phenolic resins, urea resins, melamine resins, epoxy resins, allyl resins and epoxide resins; polycarbonates; polyacrylonitriles; polyvinylpyrrolidones (cross-linked and otherwise); polymers and copolymers of vinyl monomers including polyvinyl alcohols, polyvinyl halides such as polyvinyl chlorides, ethylene-vinylacetate copolymers (EVA), polyvinylidene chlorides, polyvinyl ethers such as polyvinyl methyl ethers, polystyrenes, styrene-maleic anhydride copolymers, styrene-butadiene copolymers, styrene-ethylene-butylene copolymers (e.g., a polystyrene-polyethylene/butylene-polystyrene (SEBS) copolymer, available as Kraton® G series polymers), styrene-isoprene copolymers (e.g., polystyrene-polyisoprene-polystyrene), acrylonitrile-styrene copolymers, acrylonitrile-butadiene-styrene copolymers, styrene-butadiene copolymers and styrene-isobutylene copolymers (e.g., polyisobutylene-polystyrene block copolymers such as SIBS), polyvinyl ketones, polyvinylcarbazoles, and polyvinyl esters such as polyvinyl acetates; polybenzimidazoles; polyalkyl oxide polymers and copolymers including polyethylene oxides (PEO); polyesters including polyethylene terephthalates and aliphatic polyesters such as polymers and copolymers of lactide (which includes lactic acid as well as d-,l- and meso lactide), epsilon-caprolactone, glycolide (including glycolic acid), hydroxybutyrate, hydroxyvalerate, para-dioxanone, trimethylene carbonate (and its alkyl derivatives), 1,4-dioxepan-2-one, 1,5-dioxepan-2-one, and 6,6-dimethyl-1,4-dioxan-2-one (a copolymer of polylactic acid and polycaprolactone is one specific example); polyether polymers and copolymers including polyarylethers such as polyphenylene ethers, polyether ketones, polyether ether ketones; polyphenylene sulfides; polyolefin polymers and copolymers, including polyalkylenes such as polypropylenes, polyethylenes (low and high density, low and high molecular weight), polybutylenes (such as polybut-1-ene and polyisobutylene), EPDM copolymers (e.g., santoprene), ethylene propylene diene monomer (EPDM) rubbers, poly-4-methyl-pen-1-enes, ethylene-alpha-olefin copolymers, ethylene-methyl methacrylate copolymers and ethylene-vinyl acetate copolymers; fluorinated polymers and copolymers, including polytetrafluoroethylenes (PTFE), poly(tetrafluoroethylene-co-hexafluoropropene) (FEP), modified ethylene-tetrafluoroethylene copolymers (ETFE), and polyvinylidene fluorides (PVDF); silicone polymers and copolymers; polyurethanes; p-xylylene polymers; polyiminocarbonates; copoly(ether-esters) such as polyethylene oxide-polylactic acid copolymers; polyphosphazines; polyalkylene oxalates; polyoxaamides and polyoxaesters (including those containing amines and/or amido groups); polyorthoesters; biopolymers, such as polypeptides, proteins, polysaccharides and fatty acids (and esters thereof), including fibrin, fibrinogen, collagen, elastin, chitosan, gelatin, starch, glycosarninoglycans such as hyaluronic acid; as well as blends and copolymers of the above.

Elastomeric polymers are particularly beneficial in some embodiments. Among the elastomeric polymers are included (a) polyolefin polymers, for example, butyl containing polymers such as polyisobutylene, (b) polyolefin copolymers, for example, polyolefin-polyvinylaromatic copolymers such as polyisobutylene-polystyrene copolymers, poly(butadiene/butylene)-polystyrene copolymers, poly(ethylene/butylene)-polystyrene copolymers, and polybutadiene-polystyrene copolymers; and (c) silicone polymers and copolymers; as well as blends thereof. Specific examples of polyolefin-polyvinylaromatic copolymers include polyolefin-polyvinylaromatic diblock copolymers and polyvinylaromatic-polyolefin-polyvinylaromatic triblock copolymers, such as a polystyrene-poly(ethylene/butylene)-polystyrene (SEBS) triblock copolymer, available as Kraton®, and polystyrene-polyisobutylene-polystyrene (SIBS) triblock copolymers, which are described, for example, in U.S. Patent No. 5,741,331, U.S. Patent No. 4,946,899 and U.S. Patent No. 6,545,097.

Additional polyolefin-polyvinylaromatic copolymers are set forth in the prior paragraph.

In some embodiments of the invention the biocompatible matrix contains a hydrophobic polymer, a hydrophilic polymer, or both a hydrophobic polymer and a hydrophilic polymer.

Examples of hydrophobic polymers from which the polymers used in the present invention can be selected include: olefin polymers and copolymers, such as polyethylene, polypropylene, poly(1-butene), poly(2-butene), poly(1-pentene), poly(2-pentene), poly(3-methyl-1-pentene-), poly(4-methyl-1-pentene), poly(isoprene), poly(4methyl-1-pentene), ethylene-propylene copolymers, ethylene-propylene-hexadiene copolymers, ethylene-vinyl acetate copolymers; styrene polymers and copolymers such as poly(styrene), poly(2-methylstyrene), styrene-acrylonitrile copolymers having less than about 20 mole-percent acrylonitrile, styrene-2,2,3,3,-tetrafluoropropyl methacrylate copolymers and olefin-styrene copolymers; halogenated hydrocarbon polymers and copolymers such as poly(chlorotrifluoroethylene), chlorotrifluoroethylene-tetrafluoroethylene copolymers, poly(hexafluoropropylene), poly(tetrafluoroethylene), tetrafluoroethylene, tetrafluoroethylene-ethylene copolymers, poly(trifluoroethylene), poly(vinyl fluoride), poly(vinyl chloride) and poly(vinylidene fluoride); vinyl polymers and copolymers, such as poly(vinyl butyrate), poly(vinyl decanoate), poly(vinyl dodecanoate), poly(vinyl hexadecanoate), poly(vinyl hexanoate), poly(vinyl propionate), poly(vinyl octanoate), poly(heptafluoroisopropoxyethylene), poly(heptafluoroisopropoxypropylene) and poly(methacrylonitrile); polymers and copolymers of acrylic acid esters, such as poly(n-butyl acrylate), poly(ethyl acrylate), poly(1-chlorodifluoromethyl)tetrafluoroethyl acrylate, poly di(chlorofluoromethyl)fluoromethyl acrylate, poly(1,1-dihydroheptafluorobutyl acrylate), poly(1,1-dihydropentafluoroisopropyl acrylate), poly(1,1-dihydropentadecafluorooctyl acrylate), poly(heptafluoroisopropyl acrylate), poly 5-(heptafluoroisopropoxy)pentyl acrylate, poly 11-(heptafluoroisopropoxy)undecyl acrylate, poly 2-(heptafluoropropoxy)ethyl acrylate and poly(nonafluoroisobutyl acrylate); polymers and copolymers of methacrylic acid esters, such as poly(benzyl methacrylate), poly(methyl methacrylate), poly(n-butyl methacrylate), poly(isobutyl methacrylate), poly(t-butyl methacrylate), poly(t-butylaminoethyl methacrylate), poly(dodecyl methacrylate), poly(ethyl methacrylate), poly(2-ethylhexyl methacrylate), poly(n-hexyl methacrylate), poly(phenyl methacrylate), poly(n-propyl methacrylate), poly(octadecyl methacrylate), poly(1,1-dihydropentadecafluorooctyl methacrylate), poly(heptafluoroisopropyl methacrylate), poly(heptadecafluorooctyl methacrylate), poly(1-hydrotetrafluoroethyl methacrylate), poly(1,1-dihydrotetrafluoropropyl methacrylate), poly(1-hydrohexafluoroisopropyl methacrylate), and poly(t-nonafluorobutyl methacrylate); polycarbonates; polyimides; polyetheretherkeones; polyamides; polyvinylaceteates; polysulfones, polyethersulfones; polyesters, such a poly(ethylene terephthalate) and poly(butylene terephthalate); polyurethanes and siloxane-urethane copolymers; and polyorganosiloxanes (silicones).

Examples of hydrophilic polymers from which the polymers used in the present invention can be selected include: polymers and copolymers of acrylic and methacrylic acid, and alkaline metal and ammonium salts thereof; polymers and copolymers of methacrylamide; polymers and copolymers of methacrylonitrile; polymers and copolymers of unsaturated dibasic acids, such as maleic acid and fumaric acid, and half esters of these unsaturated dibasic acids, as well as alkaline metal or ammonium salts of these dibasic adds or half esters; polymers and copolymers of unsaturated sulfonic acids, such as 2-acrylamido-2-methylpropanesulfonic acid and 2-(meth)acryloylethanesulfonic acid, and alkaline metal and ammonium salts thereof; polymers and copolymers of methacrylate esters with hydrophilic groups such as 2-hydroxyethyl methacrylate and 2-hydroxypropylmethacrylate, polymers and copolymers of polyvinyl alcohol, which may contain a plurality of hydrophilic groups such as hydroxyl, amido, carboxyl, amino, ammonium and sulfonyl ($-SO_3$) groups; polymers and copolymers of polyalkylene glycols and oxides such as polyethylene glycol, polypropylene glycol, polyethylene oxide and polypropylene oxide; polymers and copolymers of vinyl compound having polar pendant groups such as N-vinyipyrrolidone, N-vinyl butyrolactam, N-vinyl caprolactam; polymers and copolymers derived from acrylamide; hydrophilic polyurethanes; polymers and copolymers of hydroxy acrylate; polymers and copolymers of vinylpyrrolidone including vinyl actetate/vinyl pyrrolidone copolymers, starches, polysaccharides including gums and cellulosic polymers such as guar, xanthan and other gums, dextrans, hydroxypropyl cellulose, methyl cellulose, carboxymethyl cellulose, collagen, gelatin, alginate, and hyaluronic acid.

It should be noted that all of the above listed polymers may also be used as the particle forming material.

In a suitable embodiment, the biocompatible, biodegradable matrix is a hydrogel. In a further embodiment the biocompatible, biodegradable matrix comprises gelatin or gelatin-alginate. In yet another embodiment, the biocompatible, biodegradable matrix comprises collagen. In yet another embodiment of the invention, the biocompatible, biodegradable matrix comprises carboxymethylcellulose. In another embodiment of the invention the biocompatible, biodegradable matrix comprises pectin.

In particular embodiments of the invention the biocompatible matrix may be in the form of a sponge, sheet, film, mesh, pledget, tampon or pad. In a suitable embodiment, the biocompatible matrix is in the form of a sponge.

In still a further embodiment of the invention the biocompatible matrix may be crosslinked. The selection of a proper crosslinking method should take into the considerations of crosslinking effect, maintaining integrity of the particle-drug complexes, and potential toxicity of the crosslinker to the subject. Various methods are known in the art for crosslinking various polymeric materials including physical methods such as ultraviolet light, severe dehydration, radiation, freezing and thawing cycles and chemical methods using various crosslinkers. Exemplary procedures for crosslinking the matrixes of the invention include: (1) contacting a gelatin slurry with 1-ethyl-3-(3-dimethylaminopropyl carbodiimide (EDC) for a period of time and under conditions sufficient to form a crosslinked matrix; (2) heating the lyophilized matrix for a period of time and under conditions sufficient to form a crosslinked matrix; (3) exposing the lyophilized matrix to an electron beam for a period of time and under conditions sufficient to form a crosslinked matrix; (4) exposing the lyophilized matrix to gamma sterilization for a period of time and under conditions sufficient to form a crosslinked matrix; and (5) exposing the lyophilized device to formaldehydr vapor to form a crosslinked. The physical characteristics of a polymer matrix can be varied significantly by the degree of crosslinking of the polymer. In a particular embodiment the matrix is crosslinked in a manner that would allow the particles to be liberated from the degradable matrix over a time period of from about several days to about several weeks.

Regardless of their composition, the biocompatible matrix of the present invention will typically meet all of the mechanical, chemical and biological requirements of the implantable device.

The term "biodegradable" is recognized in the art, and includes polymers, compositions and formulations, such as those described herein, that are intended to degrade during use. Biodegradable polymers typically differ from non-biodegradable polymers in that the former may be degraded during use. In certain embodiments, such use involves *in vivo* use, such as *in vivo* therapy, and in other certain embodiments, such use involves *in vitro* use. In general, degradation attributable to biodegradability involves the degradation of a biodegradable polymer into its component subunits, or digestion, e.g., by a biochemical process, of the polymer into smaller, non-polymeric subunits. In certain embodiments, two different types of biodegradation may generally be identified. For example, one type of biodegradation may involve cleavage of bonds (whether covalent or otherwise) in the polymer backbone. In such biodegradation, monomers and oligomers typically result, and even more typically, such biodegradation occurs by cleavage of a bond connecting one or more of the subunits of a polymer. In contrast, another type of biodegradation may involve cleavage of a bond (whether covalent or otherwise) internal to a side chain or that connects a side chain to the polymer backbone. For example, an antineoplastic taxane or other chemical moiety attached as a side chain to the polymer backbone may be released by biodegradation. In certain embodiments, one or the other or both generally types of biodegradation may occur during use of a polymer. As used herein, the term "biodegradation" encompasses both general types of biodegradation.

The degradation rate of a biodegradable polymer often depends in part on a variety of factors, including the chemical identity of the linkage responsible for any degradation, the molecular weight, crystallinity, biostability, and degree of cross-linking of such polymer, the physical characteristics of the implant, shape and size, and the mode and location of administration. For example, the greater the molecular weight, the higher the degree of crystallinity, and/or the greater the biostability, the biodegradation of any biodegradable polymer is usually slower. The term "biodegradable" is intended to cover materials and processes also termed "bioerodible".

In certain embodiments, polymeric formulations of the present invention biodegrade within a period that is acceptable in the desired application. In certain embodiments, such as in vivo therapy, such degradation occurs in a period usually less than about five years, one year, six months, three months, one month, fifteen days, five days, three days, or even one day on exposure to a physiological solution with a pH between 6 and 8 having a temperature of between 25 and 37°C. In other embodiments, the polymer degrades in a period of between about one hour and several months, depending on the desired application.

In embodiments of the invention the biocompatible matrix will degrade over a period of time to release the bioactive complex over a period of time. In a further embodiment the biocompatible matrix will degrade over a period of about several hours to about 1 year. In a suitable embodiment the matrix will degrade over a period or about several days to about several weeks.

In other embodiments of the invention the biocompatible matrix is shapeable. The term "shapeable" as used herein means that the matrix material can be formed or molded into a particular shape. The term shapeable shall be considered synonymous with conformable, manipulable and moldable. In embodiments of the invention the biocompatible matrix may be shaped prior to implantation of the device or may be shaped to conform to the contours of a particular organ or biological surface at the time of implantation.

The present invention includes embodiments containing pharmaceutically acceptable additives, carrier and/or excipients. Such additives, carriers or excipients may include, adjuvants, coatings colourants, buffers, binders, lubricants, disintegrants, stabilizers, and the like.

The term pharmaceutically acceptable carrier recognized in the art, and includes, for example, pharmaceutically acceptable materials, compositions or vehicles, such as a liquid or solid filler, diluent, excipient, solvent or encapsulating material, involved in carrying or transporting any subject composition from one organ, or portion of the body, to another organ, or portion of the body. Each carrier must be "acceptable" in the sense of being compatible with the other ingredients of a subject composition and not injurious to the patient. In certain embodiments, a pharmaceutically acceptable carrier is non-pyrogenic. Some examples of materials which may serve as pharmaceutically acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and waxes; (9) oils, such as peanut oil, cottonseed oil, sunflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) phosphate buffer solutions; and (21) other non-toxic compatible substances employed in pharmaceutical formulations.

Other substances can also be added to the biocompatible matrix. Plasticizers such as propylene glycol or glycerine can be included within the matrix at up to about 30% by weight of the matrix. The addition of a plasticizing agent will enhance the flexibility and strength of the final product.

Prior to implantation the device to the subject, it may be sterilized. Sterilization can be achieved by, for example, heat or E-beam sterilization. Biocompatible wetting agents will typically be incorporated into or coated onto the matrix composition prior to the sterilization procedure. E-beam sterilization of a cross-linked gelatin composition is described in U.S. Provisional Patent Application Serial No. 60/275,391, filed Mar. 12, 2001, entitled "Methods for Sterilizing Cross-Linked Gelatin Compositions", which application is incorporated herein by reference in its entirety. Heat sterilization of a cross-linked gelatin composition is described in U.S. Pat. No. 2,465,357.

The device may also be treated with a radiopaque coating deposition or ion beam surface-texturing prior to implantation. Radiopaque materials stop x-rays, making a treated device visible on an x-ray or fluoroscopic image. Various radiopaque materials can be deposited as dense, well-adhered thin-film coatings in a variety of patterns such as marker bands and stripes. Both radiopaque coating application and ion beam surface texturing can be applied to polymers. Ion beam texturing produces specific morphologies on polymer surfaces, and a variety of uniformly or randomly spaced structure types can be produced. Ion beam-textured surfaces are durable and cannot delaminate because they are an intrinsic part of the underlying surface.

The implantable device of the invention may be implanted into a subject in need thereof. The device may be implanted using minimally invasive procedures such as laparoscopy or mediastinoscopy. In another embodiment the implantable device may be implanted during a diagnostic procedure such as during a biopsy or more specifically during a lymph node biopsy. In such cases treatment may be started immediately which may improve the subjects prognosis. In another embodiment, the implantable device may be implanted during surgery. In a particular embodiment the implantable device may be implanted during surgery to excise a tumor.

The implantable device of the present invention offers the benefit of controlled release of the bioactive agents. The device of the present invention provides multiple opportunities to control the release rate of the bioactive agent. First the biocompatible biodegradable matrix can be selected such that the degradation of the matrix provides for the release of the microparticles over a desired period of time. This degradation rate can be controlled by the selection of particular polymers or co-polymers, the degree of crosslinking as well as additives which may increase or decrease the rate of degradation. Second the micro- or nanoparticles of the invention can be prepared such that the active agent is released over a prescribed time period. The degradation rate of the particles can be controlled by the selection of the particle forming material and by the addition of additives. In a further embodiment the biodegradable matrix may contain bioactive agent in particle form as described as well as free bioactive agent that is released directly upon degradation of the matrix. This embodiment could provide a bolus of bioactive agent upon degradation of the matrix followed by a period of sustained release from the microparticles.

In another embodiment of the invention the implantable device can be used to improve the pharmicokinetic profile of a bioactive agent. In a particular embodiment the implantable device of the invention can be used to increase the lethal dose ($LD_{50}$) and the maximum tolerated dose (MTD) thereby improving the safety profile of the bioactive agent. In a further embodiment the implantable device of the invention can be used to decrease the effective dose ($ED_{50}$) of a bioactive agent thereby increasing the therapeutic index ($LD_{50}/ED_{50}$) and improving the efficacy of the bioactive agent. For example a device of the present invention may be prepared which comprises particles that are of a suitable size for selective targeting of the lymphatic system. The matrix and the particle forming material can be selected to provide preferred controlled release properties. The device can be implanted at or near the site of desired treatment where the matrix will degrade releasing particles that are selectively targeted to the desired site. The particles having reached the desired site can then release the bioactive agent in a controlled manner to provide a sustained concentration of the bioactive agent over a period of time. In this manner the concentration of bioactive complex may be maintained in the therapeutic window for longer periods of time and the bioactive complex can be directed to site where it is needed thereby avoiding the potentially toxic effects of systemic administration.

The implantable delivery device of the present invention will be advantageous for many applications including:

(1) Imaging and visualization modalities of the lymphatic system such as Gamma Scintigraphy, Positron Emission Tomography (PET), Single Photon Emission Computer Tomography (SPECT), Magnetic Resonance Imaging (MRI), X-ray, Computer Assisted X-ray Tomography (CT), near infrared spectroscopy, and ultrasound. These techniques provide information regarding detection of neoplastic involvement, particularly of inaccessible nodes in subjects with malignant diseases and can also be applied in other infectious and inflammatory conditions. Knowledge on the size of the node and the filling of nodes can also be instructive. The particles so directed to the lymph nodes in diagnostic applications will contain suitable contrast or imaging agents such as ferromagnetic materials such as iron oxide, perfluorochemicals such as perfluorooctylbromide, dyes, or gamma emitting radiolabels such as Technetium-99m, Indium-111, Gallium-67, Thallium-201, Iodine-123, 125, or 131, positron emitting radiolabels such as Fluorine-18. Other imaging agents include, fluorescence emitters, photoactivated dyes

(2) Radiation therapy. Particles so directed to the lymph nodes in radiation therapy include radionuclide labeled colloids such as Gold-198 and Yttrium-90. This includes colloids with radiosensitizers, radioprotectors, or photodynamic agents. See, for example, Coleman, Int J Radiation Onc, 42(4):781 783, 1998. These could also include neutron capturing agents such as Boron-10. These agents are activated after irradiation with neutrons. See Barth et al, Cancer Res 50:1061 1071, 1990.

(3) The delivery of therapeutic agents to lymph nodes. See, for example, Kohno et al., J Infect Chemother, 4:159 173, 1998; Lasic et al., in Vesicles, Rosoff (ed), Marcel Dekker, New York, 477 489, 1996; Bookman, Curr Opin Oncol 7(5):478 484, 1995; Alving, J Immuno Methods, 140:1 13, 1991; Daemen, in Medical Applications of Liposomes, Lasic and Papahadjopoulos (eds), Elsevier Science B.V., 117 143, 1998, Gregoriadis et al., in Medical Applications of Liposomes, Lasic and Papahadjopoulos (eds), Elsevier Science B.V., 61 73, 1998; and Woodle et al., in Medical Applications of Liposomes, Lasic and Papahadjopoulos (eds), Elsevier Science B.V., 429 449, 1998.

The implantable device of the present invention may be administered by implantation in the pleural cavity or the peritoneal cavity or in a subcutaneous compartment or vaginally or rectally.

The implantable device may be used in the treatment of a disease or condition selected from neoplasia, bacterial infection, microbial infection and viral infection. Specific examples of such diseases or conditions include, but are not limited to, lung cancer, ovarian cancer, esophageal cancer, breast cancer, colorectal cancer, prostate cancer, gastrointestinal cancer, hepatic cancer, pancreatic cancer, head and neck cancer, skin cancer, lymphoma, sarcoma, thymoma, mesothelioma, lymphatic metastases, filariasis, brucellosis, tuberculosis and HIV infection. The disease or condition may be lung cancer or lymphatic metastases of lung cancer.

In a further embodiment of the invention a process is provided for preparing an implantable device of the invention comprising combining a bioactive agent and a particle forming material in a suitable solvent to form a solution or suspension, then spray drying the solution or suspension to form particles of the bioactive complex. The particles of the bioactive complex are then combined with a biocompatible matrix in a suitable solvent. The solvent is then removed resulting in an implantable device of the invention.

The term "neoplasia" as used herein refers to an abnormal, disorganized growth in a tissue or organ, usually forming a distinct mass. Such a growth is called a neoplasm. Neoplasia is understood to include such terms as cancer, tumor and growth and may be benign or malignant.

The term an "effective amount" or a "sufficient amount" of an agent as used herein is that amount sufficient to effect beneficial or desired results, including clinical results, and, as such, an "effective amount" depends upon the context in which it is being applied. For example, in the context of administering an agent that treats cancer, an effective amount of an agent is, for example, an amount sufficient to achieve such a treatment as compared to the response obtained without administration of the agent. Administration of an effective amount of a bioactive agent is defined as an amount effective, at dosages and for periods of time necessary to achieve the desired result. For example, an effective amount of a substance may vary according to factors such as the disease state, age, sex, and weight of the individual, and the ability of antibody to elicit a desired response in the individual. Dosage regime may be adjusted to provide the optimum therapeutic response.

As used herein, and as well understood in the art, "treatment" is an approach for obtaining beneficial or desired results, including clinical results. Beneficial or desired clinical results can include, but are not limited to, alleviation or amelioration of one or more symptoms or conditions, diminishment of extent of disease, stabilized (i.e. not worsening) state of disease, preventing spread of disease, delay or slowing of disease progression, amelioration or palliation of the disease state, and remission (whether partial or total), whether detectable or undetectable. "Treatment" can also mean prolonging survival as compared to expected survival if not receiving treatment.

"Palliating" a disease or disorder means that the extent and/or undesirable clinical manifestations of a disorder or a disease state are lessened and/or time course of the progression is slowed or lengthened, as compared to not treating the disorder.

The implantable device of the present invention may further be used in a method of imaging or visualizing the lymphatic system using gamma scintigraphy, Positron Emission Tomography (PET), Single Photon Emission Computer Tomography (SPECT), Magnetic Resonance Imaging (MRI), X-ray, Computer Assisted X-ray Tomography (CT), near infrared spectroscopy or ultrasound, comprising administering the implantable device of the present invention to a subject and performing gamma scintigraphy, Positron Emission Tomography (PET), Single Photon Emission Computer Tomography (SPECT), Magnetic Resonance Imaging (MRI), X-ray, Computer Assisted X-ray Tomography (CT), near infrared spectroscopy, or ultrasound to image or visualize the lymphatic system. In a particular embodiment the particles so directed to the lymphatic system in diagnostic applications will contain bioactive agents that are suitable contrast or imaging agents such as ferromagnetic materials such as iron oxide, perfluorochemicals such as perfluorooctylbromide, dyes, or gamma emitting radiolabels such as Technetium-99m, Indium-111, Gallium-67, Thallium-201, Iodine-123, 125, or 131, positron emitting radiolabels such as Fluorine-18.

The implantable device of the invention may be useful for visualization or imaging the sentinel lymph node in neoplastic diseases. The sentinel lymph node is the first lymph node encountered by a metastasizing tumor cell after it has entered the lymphatic system. The importance of the sentinel lymph node lies in the fact that metastasizing tumor cells are recognized by the immune system and stopped there. Many times, these tumor cells are destroyed by the immune cells located in the sentinel lymph node. However, tumor cells can survive, creating a foci of metastatic disease in the sentinel lymph node. If tumor cells have metastasized to other locations in the body, malignant tumor cells will be found in the sentinel lymph node 99% of the time. On the other hand, if no tumor cells are found in the sentinel lymph node after close pathological examination, it is very unlikely that the cancer will reoccur after the primary tumor has been removed for these reasons, it is very important to locate the sentinel lymph node and, if necessary, target treatment specifically to it. The present invention provides a new approach for delivery bioactive agents, such as radiolabelled colloids, or other detectable colloids to the sentinel lymph nodes. The implantable device of the invention can be introduced into the specific site or location through minimally invasive procedure prior to the surgery. Then the sentinel lymph node can be identified during the surgery, with careful pathologic examination, to determine the extent of the lymph node dissection.

The new formulation of PLGA-PTX described herein avoids using Cremophor EL which is contained in the current injectable paclitaxel formulation. Cremophor EL can cause severe anaphylaxis. Accordingly, the implantable device of the present invention is advantages as it avoids using necessary but undesired additives that are found in i.v. formulations.

The articles "a" and "an" are used herein to refer to one or to more than one (i.e., to at least one) of the grammatical object of the article.

The terms "comprise" and "comprising" are used in the inclusive, open sense, meaning that additional elements may be included.

The following non-limiting examples are illustrative of the present invention:

## EXAMPLES

### Abbreviations

| | |
|---|---|
| DCM | dichloromethane |
| DMSO | dimethyl sulfoxide |
| DOX | doxorubicin |
| EDC | 1-ethyl-3-(3-dimethyl aminopropyl) carbodiimide |
| FBS | fetal bovine serum |
| GFP | green fluorescence protein |
| HPESO | hydrolyzed polymer of epoxidized soybean oil |
| HPLC | high performance liquid chromatography |
| NHS | N-hydroxy succinimide |
| PBS | phosphate buffered saline |
| PLGA | poly(D,L-lactide-co-glycolide) |
| PLM | polymer-lipid microparticle |
| PLN | polymer-lipid nanoparticle |
| PTX | paclitael |
| PVDF | polyvinylidene fluoride |
| PVP | polyvinylpyrrolidine |
| RPMI | Roswell Park Memorial Institute |
| SDS | sodium dodecyl sulfate |
| SEM | scanning electron microscopy |
| TEM | transmission electron microscopy |

### Chemicals

Poly(DL-lactide-co-glycolide) (PLGA, 75:25, MW 90000-126000), gelatin (type A 275 Bloom), sodium alginate, paclitaxel (taxol or PTX), collagenase, 1-ethyl-3-(3-dimethylaminopropyl) carbodiimide (EDC), N-hydroxysuccinimide (NHS), rhodamine, sodium dodecyl sulfate (SDS), sodium azide, Polyvinylpyrrolidone (PVP, K29-32 grade, average MW 58,000), Doxorubicin (as a hydrochloride salt), stearic acid were purchased from sigma Chemical Co., Canada. Activated charcoal (USP grade) was purchased from Xenex, USA. HPESO (hydrolyzed polymer of epoxidized soybean oil) anionic polymer was kindly provided by Drs. Z. Liu and S. Erhan (Food and Drug Administration, USA) and Pluronic F68 (nonionic block copolymer) was a gift from BASF Corp. (Florham Park, NJ, U.S.A). Aminopolystyrene particles (0.29 $\mu$m, 2.18 $\mu$m, 11.2 $\mu$m) and polystyrene latex (2.5% solid particle with a mean diameter of 90 nm) incorporating fluorescein isothiocyanate (PSF) were obtained from Polysciences (Wilmington, PA). The organic solvent dichloromethane (DCM) was HPLC solvent. Acetonitrile used as mobile phase in high performance liquid chromatography (HPLC) was HPLC grade. Stearic acid was recrytallized in 95% ethanol for purification. Phosphate buffered saline with calcium and magnesium which was also purchased from Sigma Chemical Co. was used as buffer solution for the *in vitro* release measurement. Distilled water produced by Millipore (Millipore Corporation, MX, USA) was used throughout. LYVE-1 rabbit polyclonal antibody (ab14917), at 1:500 dilution in Dako antibody diluent was obtained from abcam.

### Cell lines and animals

NCI-H460 human large cell lung carcinoma cells were obtained from the American type Culture Collection (Rockville, MD). The cells were grown in T80 flasks in RPMI 1640 containing 10% fetal bovine serum without antibiotics in a 95% air/5% $CO_2$ atmosphere at 37°C. Cells were sub-cultured when they reached approximately 80% confluence.

DLD-1 human colon cancer cell line transfected with hepatic growth factor receptor Met, expressing green fluorescence protein (GFP) was kindly provided by Dr. MS Tsao (Ontario Cancer Institute, Toronto, Canada). The cells were routinely cultured in Dulbecco's modified Eagle medium (DMEM) supplemented with 10% fetal bovine serum (FBS) (Gibco-BRL, Grand Island, NY).

4 week old male Rowett nude rats (CR:NIH-RNU) and female Sprague Dawley rats weighing 200-250 g were obtained from Charles River Laboratories Inc. Upon arrival animals were maintained under specific pathogen-free conditions in microisolator cages in an isolated colony under controlled light, temperature, and humidity. Animals acclimated for 1-2 weeks before the start of study protocols. Female Severe Combined Immune Deficient (SCID) mice were bred in house at Ontario Cancer Institute (Ontario, Canada). The animals were used for the experimental study at age of 6-8 weeks. All animals were fed autoclaved food and water *ad libitum*. All manipulations were carried out under sterile conditions in a laminar flow hood. The health condition of the animals was assessed regularly. Animals were humanely euthanized by $CO_2$ asphyxiation when they showed evidence of advanced cachexia or impending death. Prior to the animal experiments, an institutional animal care approval was obtained from University Health Network/Ontario Cancer Institute, University of Toronto.

**Example 1: Intrapleural lymphatic distribution of various species of particulates**

Investigation of intrapleural lymphatic distribution of various species of particulates has been conducted in rat models (Liu J et al Lung Cancer 2006;51(3):377-86). The model systems consist of healthy rats, rats following pneumonectomy, and rats bearing orthotopic lung cancer to simulate clinically relevant scenarios. Particle suspension was administered into the pleural cavity for investigation. Activated carbon (charcoal) particles were first used as a tracer to demonstrate lymphatic distribution of particulates following intrapleural administration. Results from macroscopic examination, light microscopy and transmission electron microscopy (TEM) showed that carbon particles with a broad size range were taken up by regional lymphatics and lymph nodes. To determine the contribution of the lung to lymphatic uptake of these particles, intrapleural administration of carbon particles was carried out immediately following complete resection of the left lung. A similar pattern of particle distribution was seen with carbon particles widely deposited in the regional lymphatics and lymph nodes. TEM photographs revealed that the predominant size of particles seen within lymph nodes was between 1-2 $\mu$m. Further studies showed that particles in this size range had better lymphatic distribution than particles with broad size range as they can be easily identified in contralateral and peritoneal lymph nodes. Furthermore, TEM revealed that some carbon particles with much smaller size were phagocytosed by macrophages and transported within the lymphatic capillaries. Subsequently, various species of particulates including both non-biodegradable fluorescence labeled polystyrene nanoparticles with or without surface modification with poly(N-isopropylacrylamide-co-methacrylic acid) and rhodamine labeled biodegradable PLGA microparticulates were studied in healthy and in an orthotopic lung cancer model. A similar distribution pattern in the regional lymphatics and lymph nodes was found regardless of the difference in the material that constitutes the particles and the surface hydrophobicity of the particles. In the orthotopic lung cancer model, particles were significantly taken up by the regional cancerous lymph nodes after intrapleural administration. However, distribution of the particles in the lung was mainly confined to the visceral pleura where pulmonary peripheral lymphatics permeate. The selective lymphatic uptake of various particulates in the lung cancer and pneumonectomy animal models suggests the potential application of this targeting strategy in lung cancer, perhaps as an adjuvant following lung cancer resection. The observation that particles were found in contralateral mediastinal lymph nodes raises the possibility of targeting lymphatic drainage areas remote from the primary exposure site.

**Example 2: Size impact on the lymphatic distribution of particles after intrapleural administration**

A major factor in lymphatic particle distribution is particle size [xxxvi]. Aminopolystyrene particles were radiolabeled to assess the size impact on particle uptake in the thoracic lymphatic system. Aminopolystryene particles of three sizes, i.e. 0.29 $\mu$m (small), 2.18 $\mu$m (medium), and 11.2 $\mu$m (large) were directly radiolabeled with [111]Indium (In). The labeling efficiency was 68.9±2.1%, 81.9±3.2%, 61.2±4.3% for small, medium and large particles respectively. The stability of $^{111}$In-aminopolystyrene was determined in both saline and plasma obtained from rats. The results show that $^{111}$In-aminopolystyrene radiolabeling is stable for at least 72h.

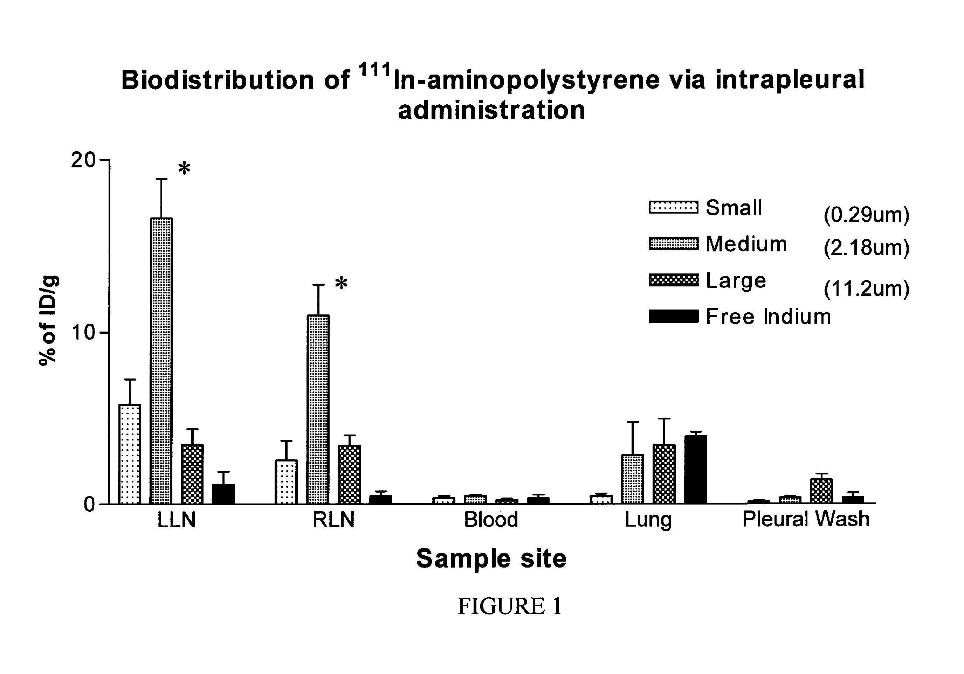

*In vivo* biodistribution study of $^{111}$In-aminopolystyrene was performed in rats with left side intrapleural administration. Four groups of rats (4/gp) were used to examine the size effect on the lymphatic uptake. Three groups of animals were treated with 0.29 $\mu$m, 2.18 $\mu$m, and 11.2 $\mu$m $^{111}$In-aminopolystyrene respectively (4mg, 200 $\mu$ci/each) and one group was treated with free $^{111}$In 200 $\mu$ci/each) as control. 24h after administration, tissue samples including left side mediastinal lymph node (LLN), right side mediastinal lymph node (RLN), blood, lung, and pleural washing fluid were collected, weighed, and subsequently counted for radioactivity. The percentage of injected dose (%ID) per organ was calculated by comparison with a standard aliquot of injected samples. The tissue biodistribution results are shown in Figure 1. 24h after intrapleural administration, the 2.18 $\mu$m polystyrene particle had significantly higher lymphatic uptake to both LLN and RLN compared to other groups. For the LLN, the %ID for the small, medium, large size groups and the control group was 5.81±2.93%, 16.64±4.58%, 3.47±1.83%, and 1.14±1.32% respectively. For the RLN, the %ID for the small, medium, large size groups and the control group was 2.55±2.28%, 10.98±3.59%, 3.41±1.19%, 0.499±0.468% respectively. (Mean±SD, n=4, P<0.01, unpaired student *t* test).

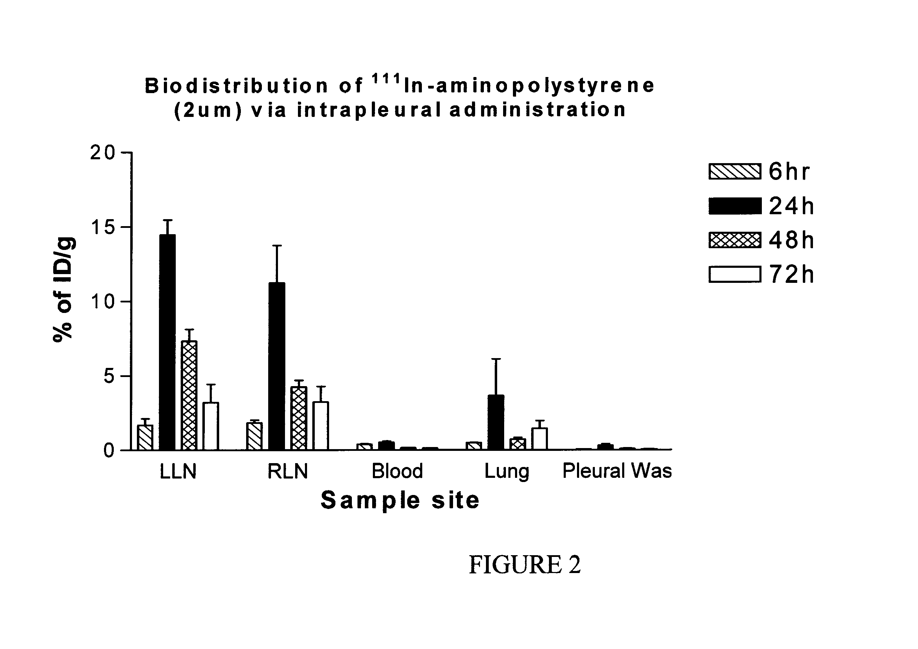

In order to further examine the particle retaining time in the regional lymphatic tissue, medium sized (2.18 $\mu$m) $^{111}$In-aminopolystyrene particles were administered intrapleurally and the *in vivo* biodistributions were investigated over 6h, 24h, 48, and 72h following intrapleural administration. The peak lymphatic uptake occurred approximately 24h after the injection. Uptake in LLN and RLN at 24h was 14.46±1.5% and 11.24±4.35% respectively. After 72h, there was 3.20±3.24% and 2.13±1.84% of ID retained in the LLN and RLN respectively. The systemic uptake into the blood, lung, was of 0.121±0.029%, 1.229±0.678% respectively, significantly lower levels than that distributed to the lymphatic tissue (p <0.01, Figure 2). Scintigraphic imaging showed that there was significant accumulation of [111]Indium radioactivity in the medistinal nodal area. This is well correlated with the autopsy finding of particle accumulation in the lymphatic tissues.

The results indicate that particle size has significant impact on particle distribution in the lymphatic system. Approximately 2 $\mu$m seems to be a suitable size for thoracic lymphatic targeting through intrapleural administration in a rat model.

## Example 3: Synthesis and characterization of PLGA, PLGA-PTX microspheres

### Preparation of PLGA and paclitaxel loaded PLGA microparticulates

PLGA microspheres were fabricated using spray dry technique according to Mu L et al.[xxxviii] with some modifications. In brief, a laboratory scale spray-drying was carried out by using the Buchi™ mini spray dryer B-191 (Buchi™ Laboratory-Techniques, Switzerland) with a standard nozzle (0.7 mm diameter) to prepare paclitaxel-loaded PLGA microspheres. The operating conditions were set as follows: inlet air temperature 54°C, outlet temperature 43°C, spray flow control (700 NL/h), pump setting at feed spray rate 4.0-4.5 ml/min, atomization pressure 6 bar (90 PSI), aspirator setting level (100%). PLGA, PTX, were dissolved in an appropriate volume of DCM, (the total concentration of the material matrix and drug in the organic solution was ~2%; 2.0 g PLGA / 100 ml DCM; PLGA : PTX = 1: 0.08 w/w), then stirred at room temperature using a magnetic stirrer until all components were dissolved homogeneously. The solution was then spray dried till no more production can be sprayed out. For making PLGA-rhodamine particles, rhodamine powders were dissolved in DCM along with PLGA polymer (20 mg rhodamine, 4.0 g PLGA in 200 ml DCM). The dried product was collected. The microspheres obtained were stored in a vacuum desiccator at room temperature. Placebo microspheres in which PTX and rhodamine was absent were prepared with the same procedure.

### Characterization of paclitaxel-loaded PLGA microparticles

### Particle size and morphology

Particle size distributions were determined using Coulter LS230 laser diffraction particle size analyzer (Beckman Coulter, Inc.). Microspheres were suspended in water with 0.1% Tween 80 to prevent aggregation prior to particle size analysis. For each sample, the mean diameter of three determinations was calculated. Values reported are the mean±standard deviation of three different batches of the particles.

The surface morphology of PLGA-PTX microparticles, raw PTX powders were determined using a scanning electron microscope (SEM) (JEOL JSM-5900LV).

### Determination of drug content in the PLGA particles

The measurement of PTX content in the microspheres was carried out in triplicate using HPLC (HP1090 liquid chromotograph). 0.5 mg of paclitaxel-loaded microspheres was accurately weighted and dissolved in 0.5 ml of DCM. A nitrogen stream was gently introduced to evaporate DCM at room temperature. Then 1.0 ml of acetonitrile-water (50:50 v/v) was added and mixed until a clear solution was obtained. The solution was put into a vial for HPLC detection. Mean values of total content of PTX were calculated from three replicates for each formulation. A reverse phase Inertsil ODS-2 column (C18) with pore size of 5 $\mu$m was used. The mobile phase consists of a mixture of acetonitrile-water (50:50, v/v), and was delivered at a flow rate of 1.0 ml/min with a pump. 50 $\mu$l aliquot of the samples was injected with an auto-injector. The column effluent was detected at 227 nm with an UV detector. The drug loading was calculated as the weight ratio of the drug entrapped in the microspheres to that of the weight of the PLGA-PTX particle compound.

Attempts were also made to determine the possible free PTX presented in the PLGA-PTX microspheres. The microspheres were collected and washed three times with distilled water to remove possible free PTX. The microspheres were collected by filtration through a cellulose nitrate membrane with pore size of 0.45 $\mu$m (Cat. No. 200-4045, Nalgene™ Labware) and dried at room temperature under reduced pressure. The fraction of PTX in the washing fluid and the fraction of the PTX remaining on the PLGA microsphere was further determined using HPLC. The obtained microspheres were kept in the desiccator at room temperature before further analysis.

### In vitro drug release of PLGA-PTX microspheres

The in vitro release of paclitaxel loaded PLGA microspheres was measured in PBS in triplicate at temperature of 37°C. 15 mg of paclitaxel loaded microspheres were suspended in 10 ml of PBS containing 0.1% (v/v) Tween™ 80 in a screw capped tubes, The tubes were tumbled end-over-end at 30 rpm at 37°C in a thermostatically controlled oven and at given time intervals, centrifuged at 10,000 rpm for 10 minutes and the top 8 ml of the supernatant was saved for analysis. The tubes were refilled with 8 ml fresh PBS. The precipitated microspheres pellets were resuspended in 10 ml of PBS buffer and placed back to the oven. The amount of paclitaxel in 8 ml of the supernatant was determined by extraction of paclitaxel into 3 ml of DCM followed by evaporation to dryness at 45°C under a stream of $N_2$, reconstitution in 2 ml of 50:50 acetonitrile in water (v/v) and analysis by HPLC as described previously. Cumulative drug release profiles were generated.

### Results

### Microsphere preparation and encapsulation efficiency

PTX loaded PLGA microspheres, PLGA-rhodamine microspheres and placebo PLGA were successfully fabricated using a spray dry technique. The concentration of PLGA in organic solvent was 2% (w/v). The yield of spray dry production was 12.3%. The drug loading of the particle ranged from 6.6%-7.2% (w/w) containing 82.5%-90% of the expected amount of initial PTX loading (8%) depending on products of different batches. The free fraction of PTX washed from the PLGA-PTX microspheres was 0.69% of the total drug loading, a negligible amount. Thus the PLGA-PTX microspheres used for the rest of the study were not subject to the procedure of washing.

The spray dry technique is widely used in the pharmaceutical and biochemical fields. It has several advantages, despite a relatively low production yield. Spray drying is a one stage continuous process, which usually produces uniformed size particles. It is easy to scale up, simpler and faster than other conventional methods used in microsphere fabrication. Several other techniques are described to formulate PLGA-PTX micro- or nanoparticles[xxxix xl xli xlii], of which the most widely used is solvent evaporation.

PTX is a highly lipophilic agent and is relatively heat resistant with a melting point of 213-220°C. These characteristics make it particularly well suited for the fabrication parameters and the solvent used in this study. Potential exists to enhance the encapsulation efficiency and drug loading by manipulating the spray dry parameters or introducing chemical additives, such as cholesterol[xliii].

### Morphological characteristics of the microspheres

SEM photomicrographs show that all three types of the microparticles have a similar spherical morphology with intact relatively smooth surfaces. The raw PTX particles exhibit an irregular crystal structure with large variation of particle sizes. SEM examination found no evidence of PTX crystals embedded on the surface of the microspheres.

### Particle size and size distribution

All three types of PLGA microspheres have a narrow size range of 1-8 $\mu$m. Three batches of PLGA-PTX particles were produced. The mean size of the microspheres was 3.5±2.1 $\mu$m. The particle size ranges were further confirmed by SEM examination. Particle size is an important factor in determining lymphatic particle distribution[xliv]. Optimal particle size for lymphatic delivery varies with different body compartments[xlv]. When particles are administered intraperitoneally or intrapleurally, the open junctions on the lymphatic wall are the only size limitation barriers[xlvi], as particle drainage is simply from cavity into the surface lymphatics without diffusion through the interstitial space. It is suggested that the suitable particle size for targeting regional lymph nodes via intraperitoneal or intrapleural administration is about 1-5 $\mu$m [xlvii xlviii], while nano size may be required to target lymphatics elsewhere[xlix]. The particle size we formulated appears suitable for lymphatic targeting through intraperitoneal and intrapleural administration. It was not our intention to formulate PLGA particles with various sizes in this study. However, PLGA particles with different size ranges can be made using different formulation methods [l li lii liii]. Application of appropriate emulsifiers or additives in the formulation can control particle size and size distribution[liv].

## *In vitro* release of paclitaxel from PLGA microparticles

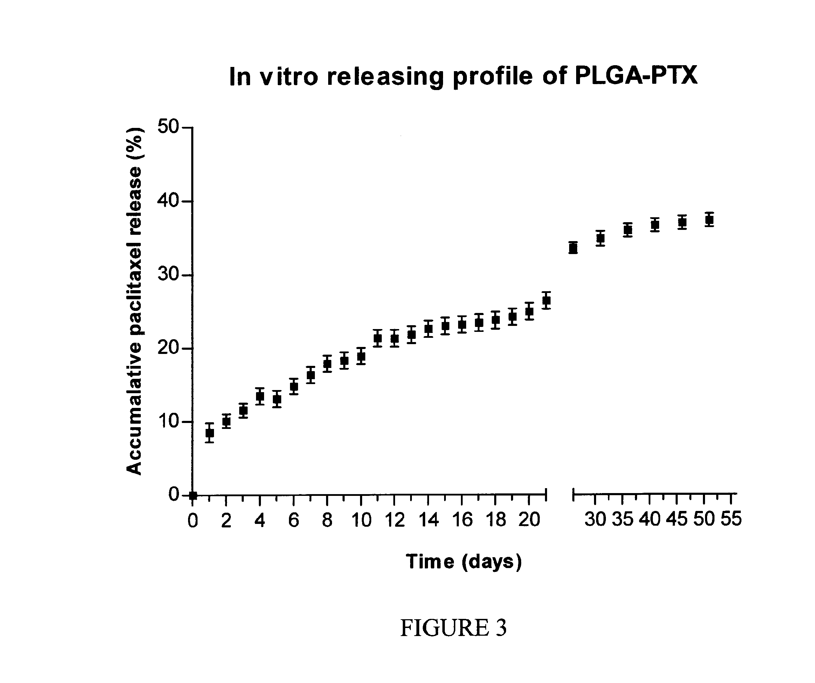

*In vitro* paclitaxel release profile from PLGA-PTX microspheres are shown in Figure 3. Sink conditions were maintained after the first 24 h of the study with the replacement of the releasing medium during each measurement. The accumulative drug release was measured daily in the first 21 days, and was extended to every 5 days for the rest of the study because of slow drug release. The microspheres release approximately 37% of the total encapsulated paclitaxel which is equivalent to 2.4 mg of paclitaxel/100mg of microspheres over the course of the release study. An initial phase of burst release was observed in the first 24 h period during which 6.8% of the loaded drug was released. This was followed by a slower phase with close to zero-order release kinetics observed between second and fourteenth days. Between days 14 and 51, the releasing rate decreased gradually.

## Example 4: Synthesis and characterization of gelatin sponge containing microparticulates

## Preparation of gelatin sponges with PTX and PLGA-PTX particles

Gelatin powders (type A 275 bloom) were dissolved in distilled water at 50°C for 2 h to prepare 2% (w/w) gel solutions. 5.0 mg PLGA-PTX, or its equivalent amount of free PTX (330 $\mu$g) were dispersed in 0.1% (w/v) Tween™ 80 dH$_2$O. The pre-prepared particle suspension was then mixed with equal volume of the 2% gel solution to make 1% (w/w) mixed gel solution. The gel solution was mixed thoroughly by gentle pipetting and 2 ml of the solution then placed into the plastic wells of a 24-well cell culture plate (Corning). The samples were frozen at -30°C for 2h and freeze-dried using a tray freeze dryer (Dura-stop $\mu$P FTS System). The freeze drying conditions were set as follow. Primary drying, at -30°C, chamber vacuum 200mT, for 72h. Secondary drying at 20°C, chamber vacuum $\pm$ 20mT, for 4h.

## Preparation of gelatin-alginate sponge containing various microspheres

Powders of gelatin and sodium alginate were dissolved in double distilled water at 50°C for 2 h to prepare 1 % (w/w) and 2 wt% (w/w) solutions. Each solution was mixed with the weight ratios of gelatin to sodium alginate to be 7:3, and 5:5 and was stirred at room temperature for 30 min. 5 mg PLGA or PLGA-rhodamine microspheres or carbon colloids (with three different size ranges: 700 to 1500 nm, 400 to 600 nm and 140 to 240 nm) were then dispersed in to 20 ml gel solution containing 0.1% Tween 80 (v/v). The gel solution was then filled into 24-well polystyrene cell culture plate (Corning) or small mold (353097 Cell culture Insert, Becton Dickinson) and was frozen at - 70°C for overnight and lyophilized at -50°C for 72 h

## Morphology of gelatin sponge containing microparticulates

The morphology of gelatin sponge impregnated with PLGA, PLGA-PTX, PLGA-rhodamine, and carbon colloids was examined using light microscopy, fluorescence microscopy and SEM accordingly.

## Preparation of crosslinked gelatin sponges containing PLGA-PTX microspheres

## Crosslinking with EDC/NHS system

The selection of a proper crosslinking method should take into the considerations of crosslinking effect, maintaining integrity of the PLGA-PTX microspheres, and potential toxicity of the crosslinker to human tissue. In the present study, the crosslinking of the gelatin sponge was carried out by using (EDC/NHS) system[lv] with some modifications. The EDC/NHS system was selected because it has shown better biocompatibility than others[lvi]. The crosslinking takes place by a reaction between carboxyl groups of glutamic or aspartic acid residues and amine groups to form amide bonds[lvii lviii]. The crosslinking reaction results in a water-soluble urea derivative as the only byproduct, which can be easily eliminated from the human body. Hence, the concern over the release of toxic residuals, commonly associated with other chemical crosslinking agents, is reduced.

EDC/NHS was directly introduced into the gelatin gel solution in two different molar ratio of EDC:COOH (1.75:1 and 7:1) to obtain the sponges with different degree of crosslinking, while the molar ratio of NHS to EDC was kept constant at 0.4. After adding the crosslinkers, the samples were maintained at 4°C for 30 minutes before the initiation of freeze drying.

**Crosslinking with formaldehyde vapor**

An alternate method of crosslinking using formaldehyde vapor was also attempted after the sponges were synthesized. This may help maintain sponge shape and texture compare to adding crosslinker directly into the gel solution or soaking the sponge into the crosslinking medium. Non-crosslinked sponges were suspended over a 1 cm deep layer of 37% aqueous formaldehyde in a closed container at room temperature for crosslinking. The exposure time of formaldehyde vapor varied from 15min to 24h. After crosslinking, the sponges were air vented and placed in a dessicator under continuous vacuum for 2h to minimize the residual aldehyde in the sponge.

**Assay of *in vitro* drug releasing**

The *in vitro* release of PTX from PLGA-PTX microspheres and gelatin sponges impregnated with free PTX or PLGA-PTX microspheres was measured in a phosphate buffer solutions (1xPBS pH 7.4) containing calcium and magnesium and 0.5% (w/v) sodium dodecyl sulfate (SDS) at a physiological concentration of bacterial collagenase (type IV) 500 ng/ml from Clostridium histolyticum (EC 3.4.24.3 Sigma Chemical Company, St Louis, MO) in quadruplicate. Microspheres or gelatin sponge sample containing equivalent amount of 330 $\mu$g PTX were submerged in 50 mL of incubation medium in 50-mL conical screw capped tubes, preserved with 0.05% (w/v) sodium azide to prevent microbial growth. The samples were incubated at 37°C and were shaken horizontally at 100 min$^{-1}$ in a shaking incubator (Multitron™ II). At given time intervals, the tubes were centrifuged at 5000 rpm for 15 min. 200 $\mu$l of supernatant was then passed through a 0.22 $\mu$m syringe-driven filter with PVDF membrane (MILLEX®-GV, Millipore, Carrigtwohill, Co. Cork, Ireland). An aliquot of 100 $\mu$l filtrate was mixed with equal volume of acetonitrile in a 1.5 ml Appendorf™ tube. After brief vortex the mixture was centrifuged at 10000 rpm for 5min. The supernatant was aliquoted for HPLC detection as described previously. The amount of PTX was calculated from the calibration curves. Cumulative drug release profiles were obtained with the correction of the volume loss. The mass balance of paclitaxel was determined at the end of the drug releasing study (Table 1) illustrates the samples selected for *in vitro* drug releasing study.

*In vitro* **degradation of gelatin sponge**

The degradation behavior of non-crosslinked and crosslinked gelatin sponge (EDC/NHS) containing PLGA placebo microspheres was examined in the aqueous medium containing collagenase (500 ng/ml), which was similar to the drug releasing medium without adding SDS. Sponge samples with similar size and weight (40-45 mg) were used for the study. Samples of non-crosslinked, low crosslinked (EDC:COOH=1.75:1) and highly crosslinked (EDC:COOH=7.1) (n=3 for each type) were immersed in 40 ml of the degradation medium respectively. The samples were incubated at 37°C and were shaken horizontally at 100 min$^{-1}$. The time need for complete degradation of the sponge was recorded. The study last for 3 weeks. The release of the PLGA microspheres was also under observation.

A modified enzymatic degradation assay was employed to assess the degradability of the gelatin sponge crosslinked with formaldehyde vapor. Pieces of sponges weighing 40-45 mg were soaked at 37°C in a PBS solution containing $CaCl_2$ and collagenase (type IV from Sigma Chemical Co.) at a concentration of 200 $\mu$g/ml (200 mg per gram of sponge). The period for the complete degradation of sponges was recorded.

**Swelling of gelatin sponge**

To measure the swelling ability of the sponge, a pre-weighed dry sponge sample was immersed in distilled water for 20 s. After the bulk water was removed by placing the wet sponge on the Petri-dish for 1 min, the weight of wet sample was measured. The procedure was repeated with 6 different pieces of sponge. Then, the sponge swelling ability was determined according to the following equation:

$$\%Swelling = [(Ww-Wd) \times 100/Wd]$$

Where Ww and Wd represent the weight of wet and dry sample, respectively.

**Results**

**Morphology of the gelatin sponge impregnated with PLGA microspheres**

Gelatin and gelatin alginate sponge devices can be prepared in different sizes and with different geometry and content. Representative sponge samples of various types are shown in Figure 4. The morphology of PLGA-PTX microspheres and the microspheres in the gelatin sponges is similar, indicating that the process of synthesizing the gelatin sponge has no significant impact on the particle morphology. All of the prepared sponges demonstrate similar lattice structures. At higher magnification the microspheres inside the sponge structure are clearly evident, where the microparticles are uniformly dispersed within the sponge network. In some areas the microspheres appear in the form of grape-like bundles intermingled with the sponge matrix. The morphology of the crosslinked gelatin sponge containing PLGA-PTX microspheres was also examined. The pore size of the sponge became enlarged when the gelatin gel was treated with EDC/NHS compared to that of the non-crosslinked gelatin sponge. However, the porosity of the crosslinked sponge was lower than that of the non-crosslinked sponge. A fluorescence micrograph of a gelatin sponge containing PLGA-rhodamine microspheres was obtained, which shows a considerable number of PLGA-rhodamine microspheres in the sponge matrices.

**Gelatin sponge-crosslinking, swelling, and *in vitro* degradation**

Crosslinking of the sponge was achieved after treating the gelatin gel solutions with EDC/NHS. The extent of the crosslinking was compared between two different molar ratios of EDC:COOH (1.75:1 and 7:1). Assessment of the mechanical properties of the sponge was not the intent of this study as the crosslinked sponge appeared more viscous. Results show that the extent of crosslinking of gelatin increases with the higher molar ratio of EDC to COOH. This resulted in a higher resistance of the sponge to degradation in PBS collagenase solution. 40 mg of the gelatin sponge made of 1% gel solution degrades in aqueous medium containing collagenase (500 ng/ml) within 45-60 min. The PLGA microspheres impregnated in the sponge were quickly released as the sponge disintegrated. The highly crosslinked gelatin sponge with molar ratio of EDC:COOH = 7:1 remained intact or insoluble in the same medium after 3 weeks of incubation. There was no obvious release of the PLGA microspheres. The relatively low crosslinked sponge with molar ratio of EDC:COOH = 1.75:1 completely disintegrated after 10 days of incubation. The release of PLGA microspheres into the medium was observed. The enzymatic degradability of gelatin sponge crosslinked by formaldehyde vapor is shown in Table 2. The results show that given the same time exposure of formaldehyde vapor, the *in vitro* biodegradability of the gelatin sponge is reversely proportional to the concentration of the gel solution. Introducing hydrophilic polymer alginate into the gel matrix tends to increase the biodegradability of the sponge. The tendency for sequestered PLGA particles to leak from gelatin matrix depends on a number of factors: degradation time, crosslinking effect, releasing medium and its composition. Therefore, the *in vitro* degradability, which partially reflects the crosslinking effect, should be interpreted according to the conditions of the assay.

Swelling of the gelatin sponge was reversely proportional to the degree of crosslinking. The percentage of swelling was $746\pm41\%$, $522\pm24\%$, and $425\pm39\%$ for non-crosslinked, low crosslinked and highly crosslinked sponges (EDC/NHS) respectively. This tendency might be attributed to the decreasing porosity of sponges by adding a crosslinker. As a result, highly crosslinked sponges could not sustain much water within their network structure.

Most gelatin sponges used for medical purposes are crosslinked to prolong their degradation in the human body. Although chemical cross-linking by several cross-linking agents, such as formaldehyde and glutaraldehyde, have been reported, the toxicity of these chemicals in biological systems becomes a great concern. EDC/NHS is a biologically safe system to crosslink gelatin sponges. The crosslinking effect can be altered by adjusting the EDC/COOH molar ratio. According to the literature, most of the crosslinking process is carried out after the gelatin sponge has been synthesized. In this case, the sponge is either exposed to aqueous medium containing EDC/NHS or to organic solvent such as 90% (v/v) acetone/water mixture containing EDC to reduce the amount of the EDC used for crosslinking. However, these approaches are not suitable for this study as they may either trigger the drug releasing or easily damage the integrity of the PLGA-PTX microspheres. Other physical crosslinking methods, such as thermal heating and ultraviolet irradiation are not appropriate either as they may damage the integrity of the PLGA microsphere or degrade PTX. The highly crosslinked gelatin sponge containing PLGA-PTX microspheres was selected for the *in vitro* drug releasing study.

**In vitro release of paclitaxel from gelatin sponge**

*In vitro* PTX release profiles are shown in Figure 5. When non-crosslinked gelatin sponges containing raw crystal PTX particles were placed in the drug releasing medium they swelled and then fully dissolved within an hour (45-60 min). The drug was quickly and near completely released (>90%) shortly after exposure to the release medium (1h) and remained at a similar level throughout the course of the study, indicating that PTX itself has no control releasing property. Since the non-crosslinked gelatin sponge disintegrated quickly in the releasing medium, the water soluble gelatin sponge has no limiting effect on PTX release into the medium. In general, the rate of PTX release from all the gelatin sponges containing PLGA-PTX microspheres was slow. The drug releasing profiles of PLGA-PTX microspheres and the gelatin sponge containing PLGA-PTX microspheres were very similar over a 19-day period. The results showed that between 7.4-8.5% of the total amount loaded was released in this time period. There was a small burst in the 1st h (approximately 2% of total loaded PTX) was released followed by a slower and continuous release. No significant difference was observed in the amount of PTX released from PLGA and gelatin-PLGA composite (non-crosslinked) at the end of the study (Day 19) (P>0.05, two-tailed *t*-test). However, the crosslinked gelatin sponge containing PLGA-PTX microspheres slowed the rate of drug release over the first three days of the drug release study. This lag phase in the initial drug release was distinct comparing to other samples (Figure 5). After a slower but steady drug release for approximately 4 day, it seemed that the PTX release reached a plateau for another 3 days. A continuous drug release followed. By day 19, there was 7.1% of the total loaded PTX released.

After the completion of the *in vitro* release study, an attempt at mass balance of the PTX was made. Total recovery of PTX calculated as the cumulative amount released *in vitro* and the amount recovered from the microspheres was 57.7%, 66.9% and 63.3% of the initial load for the PLGA-PTX, the non-crosslinked gelatin sponge containing the PLGA-PTX and the crosslinked gelatin sponge containing the PLGA-PTX respectively. The percentage of cumulative PTX release was calculated with the consideration of the final mass balance. The overall results indicate that both PLGA-PTX and sponge PLGA-PTX composites have control releasing properties. However, the control release of PTX is mainly governed by the degradation of PLGA polymer microspheres. The crosslinking of gelatin sponge may exert additional limiting effect on PTX release by retaining PLGA-PTX microspheres in its swollen matrix, which may reduce the polymer exposure to the aqueous medium. The overall cumulative PTX release obtained from this study was lower than that reported in the literature[lix lx lxi] mainly because a distinct drug releasing system and a unique sampling method was used. With this method a small volume of the releasing medium was removed for HPLC assay in distinction to other methods that replace the sampled medium with fresh releasing medium that may alter the drug releasing environment. In addition, the samples were passed through a syringe filter with a pore size of 0.22 $\mu$m (PVDF membrane with low PTX absorption) to eliminate possible contamination of microspheres for PTX detection.

Control release of PTX has strong clinical implication for treating cancer as it provides a possibility of maintaining a therapeutic drug concentration for an extended period of time. PTX acts by stabilizing microtubules to promote microtubule assembly in cells, which blocks in the $G_2$/M phase of the cell cycle[lxii]. This cell cycle-specific effect may result in a schedule-dependent effect on cytotoxicity. In particular, longer durations of PTX exposure may allow a greater proportion of cells to enter the $G_2$/M phase, which may increase cytotoxicity[lxii] demonstrated that above a PTX concentration of 0.05 $\mu$M, cytotoxicity was more dependent on increasing durations of exposure than increasing PTX concentrations[lxiii]. Also, resistance to PTX *in vitro* can be overcome by prolonged drug exposure[lxiv].

The composite of gelatin and PLGA-PTX microspheres developed in this study provides opportunities to further optimize the control release of PTX. It can be inferred from the preliminary results that by further manipulating the crosslinking effect, or by incorporating PLGA-PTX microspheres together with free PTX in appropriate composition, potential exists to achieve a desired control release of PTX for lymphatic targeting.

**Example 5: Targeting regional lymphatic system by implantation of gelatin sponge containing micro and nanoparticulates**

**In vivo lymphatic targetability**

The lymphatic targetability and biocompatibility was further investigated through both intraperitoneal and intrapleural implantation of the sponge devices in healthy rats and nude rats bearing orthotopic lung cancer. The non-crosslinked gelatin sponge containing PLGA-rhodamine microspheres and carbon colloids were applied. The sponges were surgically introduced into peritoneal cavity and pleural space of the rats. For the procedure of intraperitoneal implantation, animals were anesthetized by inhalation of 3% isoflurane. A 1.0 cm incision was made at upper gastric area of the abdomen under sterile conditions. A gelatin sponge weighting 40 mg, containing PLGA-rhodamine microspheres was inserted into the peritoneal cavity at the site of lesser curvature of the stomach which is rich in lymphoid tissue. The abdominal incision was closed with surgical wound clips.

For the procedure of intrapleural implantation, the animals were initially anesthetized by using an isoflurane induction chamber with ~5% isoflurane and were then intubated through mouths with 16-gauge angiocatheters (Becton Dickinson, Infusion therapy systems Inc. Sandy, Utah 84070). The animals were placed in a right lateral decubitus position with restraint of the limbs. The catheter was connected to a volume-cycled rodent ventilator delivering a tidal volume of 10 ml/kg at a rate of 80-100 breaths/min and maintaining 3% isoflurane. The left side of the chest wall was cleaned, shaved, and sterilized with betadine. A 1.5 cm anterolateral skin incision was made over the fifth intercostal space and extending into the pleural cavity. The ribs were held apart by a sterile self-retaining retractor. The pre-prepared gelatin sponge containing PLGA-rhodamine or carbon colloids was introduced into the medial side of the pleural space. After the sponge insertion, the ribs were reapproximated with 4-0 silk suture and the skin incision was closed with surgical wound clips. The similar intrapleural implantation was carried out in a well established H460 orthotopic lung cancer model[lxv]. The sponge containing 5mg of PLGA-rhodamine was introduced into the pleural cavity through left thoracotomy at 21 days following endobronchial implantation of H460 lung tumor.

Animals were euthanized at 3 or 7 days following the implantation (n=3 for each group). Interested tissue especially regional (mediastinal, celiac) lymph nodes, other lymphoid tissues, omentum, mesentery were harvested for microscopic examination. The tissue specimens were placed in tissue wells filled with LAMB ornithine carbamyl transferase (OCT) embedding medium and were rapidly frozen on dry ice. 3 $\mu$m serial tissue cryosections were made for both fluorescence and light microscopic detection. After identification of the fluorescence labeled particles, the paired tissue slide underwent H&E staining for confirmation of the histology by light microscopy. The surgical areas were examined for evidence of degree of intactness and residual pieces of the sponges.

## Results

### *In vivo* assessment of lymphatic targetability of gelatin PLGA composites

There were no complications resulting from sponge implantation in animals. Three days after the implantation, all sponges were partially disintegrated. Seven days following the implantation, the sponges were almost completely disintegrated. The fluorescence microscopy revealed that PLGA-rhodamine microspheres were spontaneously taken up by the regional lymph nodes through both intraperitoneal and intrapleural implantation. Intraperitoneal implantation of gelatin-PLGA sponge also resulted in the lymphatic particle absorption by the mesentery, and posterior peritoneal lymphoid tissue, sites where cancer spread often involves. PLGA-rhodamine particles were also successfully delivered to the cancerous mediastinal lymph node through intrapleural implantation. Intrapleural implantation of carbon gelatin sponge delineated lymphatic distribution of carbon colloids to thoracic regional lymphatic tissue including ipsilateral and contralateral mediastinal lymph nodes, hilar lymph node, subcarinal lymph node, internal mammary artery lymph node and posterior mediastinal lymphoid tissue. Carbon stained celiac lymph node was also recovered through this approach. The carbon particles in smaller size range (140-240 nm or smaller) tend to be taken up by the lymphatics determined by SEM. Although the non-crosslinked gelatin sponge disintegrated quickly *in vitro* (within 1 h), a slower process of biodegradation was observed *in vivo*. The intriguing results from the preliminary *in vivo* study is encouraging as it proves the principle that both biodegradable and non-biodegradable particulate drug carriers can be effectively delivered to the targeted lymphatics and lymph nodes (local, regional and remote) through implantation of our newly developed lymphatic drug delivery device.

## Example 6: *In vitro* efficacy of PLGA-paclitaxel

H460 lung cancer cells were plated at appropriate density in 6-cm plates in RPMI-1640 medium with 10% fetal bovine serum, and allowed to attach overnight. H460 cells were treated with one of free paclitaxel, PLGA placebo or PLGA-paclitaxel at different concentrations to assess the cytotoxicity. Paclitaxel, PLGA-paclitaxel compound equivalent and PLGA vehicle were solubilized in DMSO (dimethyl sulfoxide, Sigma), and further diluted with RPMI-1640 medium. The cells in the plates were treated with the drug at concentration of 0.1-1000 n*M*. The final DMSO concentration in the drug medium was < 0.1 %. Sham treatment with this concentration of DMSO was implemented for H460 control cells. Cells were incubated at 37°C for 48h after drug addition. Drug medium was then aspirated, and cells were rinsed once with PBS. A 5 ml volume of drug free medium was then added. Cell cultures were incubated for 14 days at 37°C in a humidified incubator with a mixture of 95% air and 5% $CO_2$, allowing viable cells to grow into macroscopic colonies. Then the medium was removed and the colonies were fixed and stained with a 0.5% solution of methylene blue in 70% ethanol. The colonies were examined by using the stereomicroscope (Leica MZ FLIII). The colony counting was analyzed using an image analysis software Image pro-plus (version 6.0), from which plating efficiencies were calculated based on the number of cells plated in the 6-cm culture dishes. The colonies on each plate were counted and the result was expressed as a percentage of the colonies formed on control plates not exposed to paclitaxel. The percentage of cell kill values for the paclitaxel treatments were plotted as a function of the drug concentration used. Surviving fraction was determined by dividing the plating efficiency of the drug-treated cells by that of cells without exposure to the drug (i.e. the control)

## Results

H460 lung cancer cells appeared to be susceptible to paclitaxel treatment *in vitro*. The responses of H460 cell to paclitaxel and PLGA-paclitaxel were concentration dependent. Within the concentration range of 1.0-1000 nM, paclitaxel and PLGA-paclitaxel exhibited similar cytotoxic effect on the colony formation of H460 cells (Figure 6A). PLGA had no additional effect on colony formation comparing to sham control (Figure 6B). The duration of exposure to the drug significantly affected taxol's potency *in vitro*. When H460 cells were treated with taxol concentration of $IC_{50}$ (5 nM)[lxvi], the cytotoxicity was more dependent on increasing durations of exposure to the drug (Figure 6C).

Paclitaxel has previously been shown to be stable in cell culture medium, by Ringel, et al.[lxvii]. The potency of the paclitaxel solutions should therefore not have diminished during the course of the cell incubation.

Paclitaxel extracted from the PLGA-PTX compound is as effective as original paclitaxel, indicating that formulation of PLGA-PTX has no adverse impact on the paclitaxel efficacy. Increasing the duration of exposure to paclitaxel allows more cells in a given sample to enter the cell-cycle phases during which paclitaxel is active. With shorter periods of exposure to the drug, a greater proportion of cells may exist entirely outside the paclitaxel-sensitive $G_2$ and M phases during the treatment interval. Paclitaxel is also found to be an effective radiation sensitizer when it is given in combination with sequential or concurrent radiotherapy because it synchronizes tumor cells in $G_2$/M phase, the most radiosensitive portion of the cell cycle. Both preclinical and clinical studies have shown the radiosensitizing effect of paclitaxel[lxviii]. Therefore, targeting delivery of paclitaxel to regional lymphatic system may improve both chemo and radiotherapeutic effect on lymphatic metastasis.

**Example 7: Synthesis of gelatin sponge containing polymer-lipid hybrid micro (PLM) or nanoparticles (PLN) encapsulated with doxorubicin**

**Preparation of PLM-Dox and PLN-Dox system**

Lipid particles of two different sizes (micrometer size PLM; nanometer size PLN) with the same composition were prepared following the procedures adopted from the study by Wong et al.[lxix]. A mixture of 100 mg stearic acid and 0.9 ml of aqueous solution containing 4.2 mg doxorubicin and Pluronic-F68 (2.5 %w/v) was warmed to 72-75°C, following by the addition of 2.1 mg HPESO polymer, an anionic polymer previously shown capable of enhancing the partitioning of doxorubicin into the lipid phase[lxx]. PLN were prepared by subjecting the mixture to five cycles of ultrasonication, with each cycle lasting 2 minutes, and dispersing the resulting submicron-size emulsion in water at 4 °C (1 part emulsion to 4 parts of water). PLM were prepared by substituting the ultrasonication step with mechanical stirring with magnetic bar. Aliquots of particles were sampled for Dox loading measurement by vis/UV-spectrophotometry and particle size determination by photon correlation spectroscopy as previously described[lxxi]. Free, unloaded doxorubicin in the particle suspensions was removed by Sephadex™ C-25, a cationic ion-exchanger that effectively binds doxorubicin[lxxii], before the particles were used for implant preparation.

The pre-prepared particle suspension was then mixed with equal volume of the 2% gel solution to make 1% (w/w) mixed gel solution. The gel solution was mixed thoroughly by gentle pipetting and 1 ml of the solution then placed into the plastic wells of a 24-well cell culture plate (Corning). The gel samples were frozen at -70°C for overnight and lyophilized at -50°C for 72 h.

The gelatin sponge containing PLM-Dox or PLN-Dox was further examined by confocal fluorescence microscopy and SEM.

**Results**

Doxorubicin loading of PLM and PLN was 3.0 ± 0.5% and 3.2 ± 0.4%, respectively (n = 3). The size distribution of the particles was measured by photon correlational spectroscopy. The average particle size of PLM and PLN were 1750 ± 45 nm and 68 ± 12 nm, respectively. The particle suspensions used for the implant preparation contained 2 % (w/v) lipid content. The doxorubicin concentrations for both types of particles were adjusted to 0.6 mg/mL for implant preparation.

A SEM micrograph of the sponge containing PLM-Dox was also obtained. The PLM-Dox and PLN-Dox were integrated into the gelatin matrix as detected by the confocal fluorescence microscopy. The PLN-Dox system alone has shown extended drug release properties as it was reported in the previous study[lxxiii]. Approximately 50% of the loaded Dox released in 4h, another 20-30% released in additional 72 h. The drug probably releases more slowly in the large size particles because of the smaller effective surface area. Given the fluorescence nature of doxorubicin, the implantable system as a whole can be applied to investigate *in vivo* lymphatic targetability in various circumstances.

**Example 8: Demonstration of therapeutic effect of PLGA-PTX gelatin sponge in controlling lymphatic tumor in an orthotopic adjuvant lung cancer model**

**Orthotopic lung cancer model and left pneumonectomy**

The therapeutic efficacy of gelatin sponge impregnated with PLGA-PTX microspheres was examined in an orthotopic lung cancer model which exhibits significant tumor relapse in regional lymph node after initial surgical extirpation of the primary lung tumor[lxxiv]. It is conceivable that with resection of the primary tumors, a different metastatic pattern might have been seen, as has been observed in other experimental tumor systems[lxxv]. Such models could also provide a relevant system for studying micrometastases and efficacy of anticancer therapy in the adjuvant setting.

H460 orthotopic lung cancer model which exhibits extensive metastatic potential simulating human non-small lung cancer was previously described in detail[lxxvi]. Briefly, the H460 cells ($1 \times 10^6$) were implanted endobronchially into the lungs of pre-irradiated nude rats (5 Gy) via a small tracheotomy incision. Three weeks following cell implantation, fresh tumor tissue was harvested from the periphery of the tumor mass and mechanically cut into 0.5 mm diameter pieces under sterile conditions. A 50 mg portion of tumor fragments was then implanted into the left side bronchus of nude rats using the similar techniques. Animals are uniformly found to have regional lymph node metastases along with substantial systemic metastases.

14 days following endobronchial tumor inoculation, the animals underwent left pneumonectomy to completely remove the primary tumors, at which time the regional lymph node metastasis has not well developed yet, to simulate surgical treatment of early stage lung cancer patients. The procedures of anesthesia and thoracotomy are the same as previously described. Following successful thoracotomy, the left hilum was identified and ligated with a 4-0 silk suture. Included in the ligature was the left main bronchus and the major blood vessels supplying left lung. The left lung was excised just distal of the ligature. The resection margin was sterilized with electro cauterization. Animals were further randomized into two groups (n=8 per group). Group I: without further treatment; Group II: intrapleural implantation of gelatin sponge containing PLGA-PTX (100mg/kg). After the treatment, the ribs were reapproximated with 4-0 silk suture and the skin incision was closed with surgical wound clips. A group of animals bearing lung cancer (n=3) were treated with blank gelatin sponge with the same intrapleural implantation procedure as sham controls to discern the effect of the gelatin matrix on the cancer recurrence. Animals were followed up to 40 days (26 days after lung tumor resection) after tumor implantation to examine the incidence of tumor recurrence, especially the tumor relapse in the lymphatic system.

**Assessment of tumor recurrence**

Careful necropsies were performed following death of the animals. The animals were evaluated for gross presentation of tumor at various sites including lymph node, contralateral lung, bone, kidney, brain, soft tissue (gum) etc. Internal organs, including lung, kidney, brain, chest wall, bone, as well as lymph nodes were removed, fixed in 10% buffered formalin and embedded in paraffin. All tissues were serially sectioned and stained with hematoxylin and eosin (H&E) for microscopic examination. Any macroscopic or microscopic tumor deposit discovered, other than the primary tumor, was considered a metastasis. Organs or tissue were counted as either positive or negative for metastasis. The primary endpoints were the incidence of lymph node metastasis and tumor burden (weight and volume) of recurrent cancerous lymph nodes if there was any. The tumor volume was calculated by the formula $0.52 \times \text{length} \times \text{width}^2$. The secondary endpoint was the incidence of systemic metastasis.

**Statistical analysis**

Lymph node weight and volume were performed using analysis of variance (ANOVA) or unpaired Student's *t*-test. Fisher's exact test was used to compare the incidence of metastasis between treatment arm and the non-treatment control arm.

**Results**

Intraoperative implantation of gelatin sponge containing PLGA-PTX significantly reduced lymphatic tumor metastasis. The incidence of lymphatic metastasis was significantly lower in the treatment group 25% (2/8) compared to the controls 100% (8/8) ($p < 0.01$). One of the recurrent lymph node metastases from treatment group was only identifiable microscopically. However, there were no differences in controlling the systemic metastases between treatment and control. The tumor burden of recurrent lymph nodes reflected by the lymph node weight and volume in the treatment group was significantly lower compared to that of the non-treatment controls. Significant amount of PLGA-PTX microspheres were microscopically identified in the lymphatic tissue retrieved from the animal with the sponge implantation.

There was no difference in the pattern of tumor recurrence between sham treated control (treated with blank gelatin sponge) versus non-treatment control.

The specific suppression of lymphatic tumor metastasis in this aggressive orthotopic lung cancer model indicates an important success in the development of a lymphatic targeting strategy. Equipped with gelatin matrix, a secondary drug transporting carrier, the PLGA-PTX microspheres can be efficiently and specifically delivered to the regional lymphatic system.

**Example 9: Demonstration of therapeutic effect in controlling lymphatic tumor metastasis and targetability in colon cancer**

**Construction of DLD1 orthotopic colon cancer model**

Subcutaneous xenografts were established prior to the orthotopic colon tumor implantation by injection of $1 \times 10^6$ DLD1 (Met) tumor cells in both flanks of 2-3 animals to generate donor tumor. Before the orthotopic implantation, subcutaneous tumors were harvested and washed with antibiotic-containing culture medium. After necrotic tissue and noncancerous tissue of the specimen were removed, tumors were cut for orthotopic implantation at an average size of $1 \text{ cm}^3$. Superficial regions of the tumors containing viable tumor tissue were used for implantation. SCID mice were anesthetized by using an isoflurane induction chamber with ~5% isoflurane, followed by maintenance at 1.5 to 3.0% isoflurane delivered via a nose-only exposure unit. The anesthetized mice were fixed on a small animal surgical board with their backs to the board by tying their legs. Implantation was performed according to the method described by Pocard and colleagues[lxxvii] with some modifications. In brief, the caecum was exteriorized through a small midline laparotomy and a piece of tumor tissue sutured to the caecal surface with a single 6/0 plorene suture, leaving the tumor tissue buried in a 'pouch' consisting of a double caecal wall on each side. After implantation, the abdominal wall was closed with stainless wound clips. This model system exhibits predominant potential of lymphatic tumor metastasis. Five weeks after tumor implantation, extensive lymphatic metastasis can be identified, which include hepatic hilum or portal lymph nodes, celiac lymph nodes, mesentery lymph node, as well as through the cephalad route to the mediastinal lymph nodes. On average, the animals succumb to the disease 70-80 days following tumor implantation.

**Treatment of lymphatic metastasis in orthotopic colon cancer model with intraperitoneal implantation of gelatin sponge containing PLM-Dox**

Seven days after the cecal tumor inoculation, 20 animals were randomly divided into two groups namely treatment and non-treatment control (n=10 per group). The treatment group received further intervention by intraperitoneally implantation of a gelatin sponge containing PLM-Dox (0.3 mg/animal or sponge), while the control group did not receive further treatment. For the sponge implantation, after successful laparotomy through the initial incision, the sponge was fragmented into several small pieces to be placed at tumor implantation site, subdiaphragmatic and hepatic hilar regions.

***In vivo* imaging**

Fluorescence signal of GFP and doxorubicin was detected immediately after animals were sacrificed using Maestro™ *in vivo* imaging system (CRi Inc. Pixel MEDIA, Inc.). An additional small number of animals (n=2 for each group) were sacrificed 35 days post tumor implantation to assess the lymphatic targetability. The system is equipped with unique multispectral imaging technology to be able to differentiate the fluorescence signal of doxorubicin from GFP and other sources.

**Macro and microscopic examination**

The experimental animals were sacrificed 47 days post tumor implantation (40 days after sponge implantation) to evaluate the effectiveness of the lymphatic targeting delivery. The body weight was recorded. Autopsy was performed and macroscopic assessment was made for the presence of primary tumor, lymph-node or distant metastases. All detected macroscopic lesions were weighed and sampled for histological examination. The lymph node volume was calculated by the formula $0.52 \times \text{length} \times \text{width}^2$. For fluorescence detection, tissue samples were embedded in ornithine carbamyl transferase (OCT) compound (Miles, Elkhart, IN), frozen on dry ice and stored at -70°C. 3 $\mu$m serial cryosections were then made for fluorescence microscopic examination (Leica TCS SP2-X1 spectral confocal and multiphoton microscope). The detection of emission wavelength for doxorubicin and GFP was 540-650 nm and 460-500 nm respectively. After identification of the fluorescence labeled particles, the parallel tissue slide underwent H&E staining for confirmation of the histology by light microscopy. In order to gain an insight of the particle delivery with regard to the distribution of the lymphatics, immunostaining of the regional lymph node and mesentery tissue with antibodies to the LYVE-1, a specific lymphatic marker and a receptor for hyaluronan expressed on lymphatic endothelium[lxxviii] (Prevo R et al. J. Biol. Chem. 2001;276:19420-19430) was carried out. Frozen tissue slides were fixed in 2% formaldehyde/PBS for 20 min and blocked for endogenous peroxidase (0.3% $H_2O_2$) and biotin (Vector lab Biotin-blocking kit) activities. All subsequent reactions were carried out at room temperature and washed in PBS buffer. Slides were incubated in primary antibody to LYVE-1 (Abcam ab14917 rabbit polyclonal, at 1:500 dilution in Dako antibody diluent) for 1 hour. Secondaries were carried out with anti-rabbit IgG-biotin conjugated linking reagent (Vector Lab) followed by Streptavidin HRP labeling reagent (Idetect Ultra HRP detection system, ID Labs inc) and NovaRed (Vector Lab) as substrate. Slides were counter stained in Gill modified Hematoxylin (Harleco) and mount in Permount (Fisher Scientific).

**Statistical analysis**

Lymph node weight, volume, and primary tumor weight were performed using ANOVA or unpaired Student's *t*-test. Fisher's exact test was used to compare the incidence of lymph node metastasis.

**Results**

Orthotopic implantation of DLD1 (Met) tumor fragment on the cecal wall resulted in 100% tumor take in SCID mice. All control animals (10/10) developed lymphatic metastasis with an average of 3 metastatic lymph nodes (ranging from 1-4) identifiable in each animal during autopsy. The lymph nodes from mesentery, hepatic hilum, subdiaphragm and mediastinum were frequently involved with tumor metastases. Whereas, the lymphatic metastasis was significantly reduced in the treatment arm as 2 out of 10 (20%) animals developed lymphatic metastasis (P<0.01). Total 3 metastatic lymph nodes were identified in these two animals. Moreover, the tumor burden of the metastatic lymph nodes measured by tumor volume was significantly lower in the treatment arm comparing to that of the control group (P<0.01). The local regional administration of PLM-Dox through intraperitoneal implantation of the sponge device also moderately suppressed the primary tumor growth (P=0.062). Microscopically, numerous fluorescence signals of doxorubicin were identified in regional and remote lymphatics and lymph nodes of treated animals significantly different from what was observed in the negative controls. The *in vivo* fluorescence imaging identified significant amount of doxorubicin signals in the posterior and superior mediastinum where metastatic lymph node was found. Fluorescence microscopic examination revealed that abundant fluorescence signals of PLM-Dox appeared in the cancerous lymph node which carries GFP. Large, LYVE-1-reactive, irregularly shaped, thin-walled lymphatics were detected in the hilum region of the regional lymph nodes while the PLM-Dox mainly appeared in the cortex and medullar parts of the lymph nodes. Immunostaining of the mesentery with LYVE-1 antibody revealed many positive staining lymphatics surrounded by mesentery lymph nodes. The fluorescence signals were found in the mesentery lymph nodes and the vicinity of the lymphatics.

**Example 10: Demonstration of lymphatic targeting delivery in lymphoma**

Like the majority of cancer chemotherapies, chemotherapeutic agents treating lymphoma are mainly given intravenously to the systemic circulation. However, the therapeutic efficacy is dramatically limited by the effect of the dose limiting toxicity of the chemoagents. This study explored an alternate route for delivery of anticancer agent to the lymphoma.

Lipid micro or nanoparticulates have been shown to distribute to the localregional lymph nodes through local administration[lxxix]. Lipid-encapsulated drug formulations may also provide advantages over traditional drug-delivery methods. For example, some lipid-based formulations provide longer half-lives *in vivo,* superior tissue targeting, and decreased toxicity. Numerous methods have been described for the formulation of lipid-based drug delivery vehicles (see, for example, U.S. Pat. No. 5,741,516, incorporated herein). Incorporation of micro or nanoparticlate drug carrier into an implantable gelatin sponge in this study may provide a new direction or modality for the local control of this disease.

Lymphomas are among the most common tumors in many strains and stocks of mice. The animal model employed in this study was B6 mice with PTEN mutation. B6 mouse is one of those with high incidences (10-50%) of lymphomas in aging animals. Most of the tumors are B-cell lymphomas of the follicular type, arising in spleen, mesenteric lymph node and/or Peyer's patches. Tumor also frequently involves mediastinum and superficial lymph nodes at neck and groin areas.

B6 mice developed lymphomas manifested by enlarged cervical lymph nodes were selected for the *in vivo* study to evaluate the lymphatic targetability with the implantation of PLM-Dox gelatin sponge. The sponge was introduced into the peritoneal cavity to examine the lymphatic delivery of PLM-Dox. The control animals received blank gelatin sponge using the same approach.

Two B6 mice bearing lymphomas were chosen for each group. The procedures of anesthesia and laparotomy were the same as described previously. Gelatin sponge weighing 40 mg, containing PLM-Dox (0.3 mg/sponge or animal) was implanted into the peritoneal cavity. The wound was closed with stainless wound clips. The animals were followed for 7 days before sacrificed for assessment of lymphatic particle distribution.

Upon sacrifice of the animal, the enlarged cervical, celiac and mediastinal lymph nodes were harvested. The tissue specimens were placed in tissue wells filled with LAMB ornithine carbamyl transferase (OCT) embedding medium and were rapidly frozen on dry ice before being stored at -70°C. 3 $\mu$m serial cryosections were made with for both fluorescence and light microscopic (H&E staining) examination.

### Results

Intraperitoneal implantation of PLM-Dox sponge resulted in spontaneous absorption of PLM-Dox microspheres to the regional celiac and mediastinal lymph nodes which presented with lymphomas. Significant amount of PLM-Dox signals were identified in the lymph nodes involved with lymphoma disease.

### Example 11: Targeting lymph node draining breast

The newly developed polymer-lipid hybrid nanoparticle containing doxorubicin complex (PLN-Dox) has been shown to enhance *in vitro* cytotoxicity towards wild-type and MDR human breast tumor cell lines *in vitro*. The PLN-Dox showed much higher *in vitro* cytotoxicity against the P-gp overexpressing cell line [lxxx lxxxi]. Integrating PLN-Dox system into a biodegradable implantable matrix e.g. gelatin sponge, may offer great potential to specifically deliver PLN-Dox effectively to the lymphatics and lymph nodes related to breast cancer.

### Animals and surgical procedures

The efficiency of the delivery of PLN-Dox to the regional lymphatic system was examined in healthy female Sprague Dawley rats by subcutaneous implantation of gelatin sponge containing PLN-Dox. The control animals received blank gelatin sponges through the same implantation procedure. The lymphatic particle distribution was further examined by Maestro™ *in vivo* imaging system and fluorescence microscopy.

Rats were anesthetized by using an isoflurane induction chamber with ~5% isoflurane, followed by maintenance at 1.5 to 3.0% isoflurane delivered via a nose-only exposure unit. A 1.5-cm incision was made on the chest slightly left of the sternum and the skin separated from the chest by gentle blunt dissection. A subcutaneous pocket was created. The gelatin sponge weighing 40 mg, containing PLN-Dox (0.3 mg/sponge or animal) (size: 50-100 nm) was implanted into the subcutaneous pocket with direct contact with the mammary fat pad. The wound was closed with interrupted silk sutures. The baseline fluorescence of doxorubicin was obtained right after the implantation procedure by Maestro *in vivo* imaging system. The animal was reexamined 24h and 3 days latter to detect the lymphatic uptake of lipid-dox in the axillary region. Upon sacrifice the animal, the lymph nodes from the axillary region were harvested. The tissue specimens were placed in tissue wells filled with LAMB ornithine carbamyl transferase (OCT) embedding medium and were rapidly frozen on dry ice before being stored at -70°C. 3 $\mu$m serial cryosections were made for fluorescence microscopic examination. After identification of the fluorescence labeled particles, the paired tissue slide underwent H&E staining for confirmation of the histology by light microscopy.

### Results

There was no adverse effect resulting from the sponge implantation. The sponge was almost disintegrated 3 days after subcutaneous implantation to the mammary fat pad. The baseline *in vivo* imaging detected the distinguishable fluorescence signal of doxorubicin only at the implantation site. However, 24h, and 3-day *in vivo* imaging of the same animal revealed that the fluorescence signal of doxorubicin was identified at both armpits with relatively higher intensity on the left side. The axillary lymph nodes obtained from the treated animals contained significant amount of PLN-Dox signal examined under fluorescence microscope. There was no distinguishable fluorescence signal detected in the lymph nodes of control animal.

The results indicated that the implantable gelatin sponge containing PLN-Dox is a practically acceptable therapeutic approach for lymphatic targeting through surgical intervention. This device can be employed in both neoadjuvant and adjuvant treatment of breast cancer. Future study using breast cancer animal model is warranted.

While the present invention has been described with reference to what are presently considered to be the preferred examples, it is to be understood that the invention is not limited to the disclosed examples. To the contrary, the invention is intended to cover various modifications and equivalent arrangements included within the spirit and scope of the appended claims.

All publications, patents and patent applications are herein incorporated by reference in their entirety to the same extent as if each individual publication, patent or patent application was specifically and individually indicated to be incorporated by reference in its entirety.

**TABLE 1**

| Samples | Sponge type | | Bioactive agent |
|---|---|---|---|
| | Gelatin solution | Crosslinking | |
| PLGA-taxol microspheres | - | - | Taxol in PLGA microspheres |
| Gelatin sponge (PTX) | 1.0% | - | Taxol (free form) |
| Gelatin sponge (PLGA-PTX) | 1.0% | - | Taxol in PLGA microspheres |
| Gelatin sponge (PLGA-PTX) | 1.0% | + EDC:COOH= | Taxol in PLGA microspheres |

**TABLE 2**

| Sponge type | Exposure time of FV | Degradation time | Particle release |
|---|---|---|---|
| 1% gelatin | 15 min | 20 min | + |
| | 30 min | 120 min | + |
| | 2 h | Insoluble (2 wk) | - |
| | 4 h | Insoluble (2 wk) | - |
| 2% gelatin | 15 min | 30 min | + |
| | 30 min | 150 min | + |
| | 2 h | Insoluble (2 wk) | - |
| | 4 h | Insoluble (2 wk) | - |
| 1% gelatin-alginate (7:3) | 15 min | 10 min | + |
| | 30 min | 30 min | + |
| | 2 h | 1 wk | + |
| | 4 h | Insoluble (2 wk) | - |

FV = Formaldehyde

FULL CITATIONS FOR DOCUMENTS REFERRED TO IN THE SPECIFICATION:

[i] . Weiss, L. et al. Int J Cancer. 1987;40(4):570-4.

[ii] . Mountain CF Chest 1977;111:1718-23.

[iii] . Kuo TH Anticancer Res 1993;13:293-7.

[iv] . Martini N J Thorac Cardiovas Surg 1995;109:120-9.

[v] . Martinez SR et al. Clin Colorectal Cancer 2005;4:320-4.

[vi] . McGuire WL Breast Cancer Res. 1987;10:5-9.

[vii] . Bergquist et al. Seminars in Nuclear Med. 1983;12:9-10.

[viii] . Strand, S. E. CRC Crit. Rev. Drug Carrier System 1989;6:211-237.

[ix] . Kaledin, V. I. et al. J Natl Cancer Inst. 1981;66:881-7)

[x] . Khato, J et al. Cancer Treat Rep. 1982;66:517-27.

[xi] . Konno, H et al. Jpn J Surg. 1990;20:424-8.

[xii] . Hagiwara, A et al. Anticancer Drug Des. 1987;1:313-21.

[xiii] . Hagiwara, A et al. Anticancer Drugs. 1997;8:666-70.

[xiv]. Karajgi, J. S. et al. J Microencapsul. 1994;11:539-45.

[xv] . Yoshimura, K et al. Gan To Kagaku Ryoho. 1996;23:1519-22.

[xvi] . Porter, C. J et al. J Pharm Sci. 1996;85:351-6.

[xvii] . Yoshikawa, H et al. Biol Pharm Bull. 1996;19:1527-9.

[xviii] . Nakamura, Y et al. Surg Today. 2002;32:335-42.

[xix]. Shinohara H. Anat Rec 1997;249(1):16-23.

[xx] Liu J et al. Lung Cancer 2006; 51(3): 377-86.

[xxi] . Shimada M Anticancer Drug Des 1993;8:249-55.

[xxii] . Hawley AE, Illum L, Davis SS. Preparation of biodegradable, surface engineered PLGA nanospheres with enhanced lymphatic drainage and lymph node uptake. Pharm Res. 1997;14:657-61.

[xxiii] . Maincent P, Thouvenot P, Amicabile C et al. Lymphatic targeting of polymeric nanoparticles after intraperitoneal administration in rats. Pharm Res. 1992;9:1534-9

[xxiv] . Tokuda K, Natsugoe S, Shimada M et al. Design and testing of a new cisplatin form using a base material by combining poly-D,L-lactic acid and polyethylene glycol acid against peritoneal metastasis. Int J Cancer 1998;76:709-12.

[xxv] . Liggins TR, D'Amours S, Demetrick JS et al. Paclitaxel loaded poly(L-lactic acid) microspheres for the prevention of intraperitoneal carcinomatosis after a surgical repair and tumor cell spill. Biomaterials 2002;21: 1959-69.

[xxvi] . Hawley AE, Illum L, Davis SS: Preparation of biodegradable, surface engineered PLGA nanospheres with enhanced lymphatic drainage and lymph node uptake. Pharm Res 1997; 14:657-61.

[xxvii] . Wong HL et al. J Pharmacol Exp Ther.2006; 317: 1372-1381.

[xxviii] . Wong HL et al. Pharm Res. 2006;23:1574-85.

[xxix] . Kennedy L, Harley RA, Sahn SA, Strange C. Talc slurry pleurodesis. Pleural fluid and histologic analysis. Chest 1995;107:1707-12.

[xxx] . Singer JJ, J.J.T. Leal. Aseptic pleuritis experimentally produced. J Thorac Surg 1941; 10:251-83.

[xxxi] . Karsner HT SC. The removal of particulate matter from the pleura. J Med Res 1920; 42:91-8.

[xxxii] . Medina LA, Klipper R, Phillips WT, Goins B. Pharmacokinetics and biodistribution of [111In]-avidin and [99mTc] -biotin-liposomes injected in the pleural space for the targeting of mediastinal nodes. Nucl Med Biol. 2004;31:41-51.

[xxxiii] . Hawley AE, Illum L, Davis SS. Preparation of biodegradable, surface engineered PLGA nanospheres with enhanced lymphatic drainage and lymph node uptake. Pharm Res 1997;14(5):657-61.

[xxxiv] . Charman, William N. Lymphatic Transport of Drugs, CRC Press. Boca Raton FL, 1992; 279-315

[xxxv]. Bettendorf U. Electronmicroscopic studies on the peritoneal resorption of intraperitoneally injected latex particles via the diaphragmatic lymphatics. Lymphology 1979;12(2):66-70.

xxxvi . Hawley A.E., Davis S.S., Illum L. Targeting of colloids to lymph nodes: influence of lymphatic physiology ad colloidal characteristics. Advanced Drug Delivery Reviews 1995; 129-48

xxxviii. J Control Release 2001;76:239-54

xxxix . (onseca C J Control Release. 2002;83:273-286

xl. Liggins R Inflamm Res. 2004;53:363-72

xli. Mo Y. J Control Release. 2005 Nov 28;108:244-62

xlii. Gupte A Int J Pharm. 2004;276:93-106

xliii. Mu L et al. J Control Release 2001;76:239-54

xliv. Hawley AE et al. Pharm Res. 1997 May;14(5):657-61

xlv. Liu J et al. Lung Cancer 2006;51:377-86

xlvi. Bettendorf U Lymphology. 1979;12:66-70

xlvii. Hawley A.E et al. Pharm Res. 1997;14:657-61) (Liu J et al. Lung Cancer. 2006;51:377-86

xlviii. Liggins RT et al. Biomaterials. 2000;21:1959-69.

xlix. Liu J et al. Lung Cancer 2006;51:377-86

l. Fonseca C J Control Release. 2002;83:273-286

li. Mu L et al. J Control Release. 2003;86:33-48

lii. Mu L et al. J Control Release 2001 ;76:239-54

liii. Mo Y et al. J Control Release. 2005;108:244-62

liv. Mu, L et al. J Control Release 2001 ;76:239-54

lv. Gilbert DL et al. J Biomed Mater Res. 1990; 24:1221-39

lvi. Kuijpers A.J et al. J Biomater Sci Polym Ed. 2000; 11:225-43

lvii. Olde Damink LH et al. Biomaterials. 1996;17:765-73

lviii. Pieper JS et al. Biomaterials. 2000; 21:581-93

lix. Liggins RT et al. Inflamm Res. 2004 Aug;53(8):363-72

lx. Mu L et al. J Control Release 2001;76:239-54

lxi. Liggins RT et al. Int J Pharm. 2001;222:19-33

lxii. Liebmann J et al. Cancer Chemother Pharmacol. 1994;33:331-9

lxiii. Liebmann JE et al Br J Cancer. 1993;68:1104-9

lxiv. Lopes NM et al. Cancer Chemother Pharmacol. 1993;32:235-42

lxv. Howard et al. Clin Exp Metastasis. 1999;17:157-62

lxvi. Yamori T et al. Jpn. J. Cancer Res. 88, 1997;1205-10

lxvii. J. Pharmacol. Exp. Ther. 1987;242:692-698

lxviii Hennequin C. Cancer Radiother. 2004;8 Suppl 1:S95-105 lxix.J Pharmacol Exp Ther.2006; 317: 1372-1381

lxx.J Pharmacol Exp Ther.2006; 317: 1372-1381

lxxi. J Pharmacol Exp Ther.2006; 317: 1372-1381

lxxii. Liu Z et al. J Control Rel. 2001 b; 77: 213-224

lxxiii. Wong H et al. J Pharmacol Exp Ther.2006; 317: 1372-1381

lxxiv. Liu J et al. 94th AACR, 2003, Toronto, Canada

lxxv. Flatmark K European J Cancer 2004;40:10 1593-98

lxxvi. Howard RB Clin Exp Metastasis 1999; 17:157-62

lxxvii European J Cancer 2004 40; 10 1593-98

lxxviii (Prevo R et al. J. Biol. Chem. 2001;276:19420-19430)

lxxix. Bin Lu et al. European J Pharm Sci 2006;28:86-95

lxxx. Wong HL et al. J Pharmacol Exp Ther.2006; 317: 1372-1381

lxxxi. Wong HL et al. Pharm Res. 2006;23: 1574-85

## Claims

1. An implantable device which is a biocompatible and biodegradable cross-linked matrix impregnated with a bioactive complex suitable for selectively targeting the lymphatic system,
   wherein
   the bioactive complex is in the form of micro or nanoparticles or a combination of micro or nanoparticles having a particle size of from 10 nm to 11.2 $\mu$m that comprise one or more particle forming materials and one or more bioactive agents, and which are suitable for delivery of the bioactive agents to the lymphatic system being of a sufficient size to enter and selectively target the lymphatic system and being able to migrate through the lymphatic vessels or be retained in the lymph nodes;
   the biocompatible and biodegradable cross-linked matrix is selected such that the degradation of the matrix provides for release of the bioactive complex over a first period of time; and
   the micro or nanoparticles of the bioactive complex are prepared such that the bioactive agent is released within the lymphatic system over a second period of time.

2. The implantable device according to claim 1, which is suitable for implantation in the pleural cavity, the peritoneal cavity, a subcutaneous compartment, vaginally or rectally.

3. The implantable device according to claim 1 or claim 2, wherein the particle size is from 10 nm to 50 nm, from 50 nm to 100 nm, from 140 nm to 1500 nm, from 1 $\mu$m to 5 $\mu$m, or from 0.3 $\mu$m to 11.2 $\mu$m.

4. The implantable device according to claim 1 or claim 2, wherein the particle size is from 50 nm to 11.2 $\mu$m.

5. The implantable device according to claim 1 or claim 2, wherein the particle size is from 0.7 $\mu$m to 2 $\mu$m.

6. The implantable device according to any one of claims 1 to 5, wherein the one or more bioactive agents is a therapeutic agent.

**7.** The implantable device according to any one of claims 1 to 6, wherein the one or more bioactive agents is selected from the group consisting of radioisotopes, photosensitizers, radiosensitizers, radioprotectors, photodynamic agents, neutron capturing agents, antigens, vaccines, DNA RNA, peptides, biological response modifiers, antimicrobial agents and anti-proliferative agents.

**8.** The implantable device according to any one of claims 1 to 7, wherein the one or more bioactive agents is selected from the group consisting of anti-proliferative agents and anti-metastatic agents.

**9.** The implantable device according to claim 8, wherein the anti-proliferative agents and anti-metastatic agents are selected from the group consisting of microtubule-stabilizing agents, alkylating agents, anti-metabolites, epidophyllotoxin, antineoplastic enzymes, topoisomerase inhibitors, procarbazine, mitoxantrone, platinum coordination complexes, biological response modifiers and growth inhibitors and haematopoietic growth factors

**10.** The implantable device according to claim 9, wherein the antineoplastic agent is selected from an anthracycline drug, vinca drug, mitomycin drug, bleomycin drug, cytotoxic nucleoside, taxanes, epothilones, discodermolide, pteridine drugs, diynenes and podophyllotoxins.

**11.** The implantable device according to claim 9, wherein the bioactive agent is selected from doxorubicin, carminomycin, daunorubicin, aminopterin, methotrexate, methopterin, dichloro-methotrexate, mitomycin C, porfiromycin, trastuzumab, 5-fluorouracil, 6-mercaptopurine, gemcitabine, cytosine arabinoside, podophyllotoxin, etoposide, etoposide phosphate, teniposide, melphalan, vinblastine, vincristine, leurosidine, vindesine, leurosine, paclitaxel, estramustine, cisplatin, carboplatin, cyclophosphamide, bleomycin, gemcitibine, ifosamide, melphalan, hexamethyl melamine, thiotepa, cytarabin, idatrexate, trimetrexate, dacarbazine, L-asparaginase, camptothecin, CPT-11, topotecan, pyridobenzoindoles, interferons and interleukins.

**12.** The implantable device according claim 11, wherein the bioactive agent is selected from paclitaxel and doxorubicin.

**13.** The implantable device according to any one of claims 1-7, wherein the bioactive agent is selected from nitrogen mustards, alkyl sulfonates, nitrosoureas, triazenes, antimetabolites, pyrimidines, purines, natural products, epipodophyllotoxins, antibiotics, enzymes, substituted ureas, methylhydrazines, adrenocorticoid suppressants, hormones and antagonists, adrenocorticosteroids, progestins, oestrogens, antioestrogens and androgens.

**14.** The implantable device according to claim 13, wherein the bioactive agent is selected from mechlorethamine, cyclophosphamide, melphatan, chlorambucil, busulphan, carmustine, lomusine, semustine, streptozocin, dacarbazine, folic acid, methotrexate, fluorouracil cytarabine, mercaptopurine, thioguanine, vinca alkaloids, vinblastine, vincristine, vendesine, etoposide, teniposide, dactinomycin, daunorubicin, doxorubicin, bleomycin, plicamycin, mitomycin, L-asparaginase, hydroxyurea, procarbazine, mitotane, aminoglutethimide, adrenocorticosteroids, prednisone, hydroxyprogesterone caproate, methoxyprogesterone acetate, megestrol acetate, diethylstilboestrol, ethinyloestradiol, tamoxifen, testosterone propionate and fluoxymesterone.

**15.** The implantable device according to anyone of claims 1-7, wherein the bioactive agent is selected from a lymphangiogenesis suppressor, an angiogenesis suppressor, a cytostatic agent, macromolecules, antioxidants, cytokines, chemokines, antisense oligonucleotides, LyP-1 peptide-coated qdots to home to lymphatics, hormones and hormone antagonists.

**16.** The implantable device according to claim 15, wherein the bioactive agent is selected from vascular endothelial growth factor C, D (VEGF-C,D) antibody, the antibody to VEGF-C,D receptor (VEGFR-3), and epidermal growth factor receptor (EGFR) antibody.

**17.** The implantable device according to any one of claims 1 to 16, wherein the particle forming material is selected from the group consisting of biocompatible polymers, lipids, liposomes, metallic particles, magnetic particles, biotin, avidin and polysaccharides.

**18.** The implantable device according to claim 17, wherein the particle forming material is selected from the group consisting of polylactic acid, polyglycolic acid (PGA), polylactic-co-glycolic acid (PLGA), poly-lactic acid (PLA), polyvinyl pyrrolidones (PVP), polylactic acid-co-caprolactone, polyethylene glycol (PEG), polyethylene oxide (PEO), polystyrene, poly lactic acid-block-poly ethylene glycol, poly glycolic acid-block-poly ethylene glycol, poly lactide-co-glycolide-block-poly ethylene glycol, poly ethylene glycol-block-lipid, poly vinyl alcohol (PVA), polyester, poly(orthoester), poly(phosphazine), poly(phoasphate ester), polycaprolactaones, gelatin, collagen, a glycosaminoglycan, polyorthoesters, polysaccharides, polyhyaluronic acid, polyalginic acid, chitin, chitosan, cellulose, hydroxyethylcellulose, hydroxypropylcellulose, carboxymethylcellulose, polypeptides, polylysine, polyglutamic acid, albumin, polyanhydrides, polyhydroxy alkonoates, polyhydroxy valerate, polyhydroxy butyrate, proteins, polyphosphate esters, polyacrylamide (PAA), and mixtures thereof.

19. The implantable device according to claim 18, wherein the particle forming material is PLGA.

20. The implantable device according to any one of claims 1 to 19, wherein the bioactive complex includes additives.

21. The implantable device according to claim 20, wherein additives are selected from adjuvants, coatings, colourants, binders, buffers, lubricants, dissintegrants, plasticizers and stabilizers.

22. The implantable device according to any one of claims 1 to 21, wherein the bioactive complex comprises microparticles of PLGA and paclitaxel or lipid and doxorubicin.

23. The implantable device according to any one of claims 1 to 22, wherein the matrix degrades over a period of several hours to about 1 year, especially from several days to several weeks.

24. The implantable device according to any one of claims 1 to 23 wherein the matrix is shapeable.

25. The implantable device according to any one of claims 1 to 24, wherein the matrix is in a form selected from the group consisting of a sponge, sheet, film, mesh, pledget, tampon and pad.

26. The implantable device according to any one of claims 1 to 25, wherein the biocompatible matrix is a hydrogel film.

27. The implantable device according to any one of claims 1 to 25, wherein the matrix is selected from the group consisting of gelatin, collagen, gelatin-alginate.

28. The implantable device according to any one of claims 1 to 27, wherein the matrix contains additives.

29. The implantable device according to claim 28, wherein the additive is radio opaque material detectable by x-ray.

30. The implantable device according to any one of claims 1 to 29, wherein one or more free bioactive agents are incorporated into the biocompatible and bioactive matrix.

31. An implantable device as defined in any one of claims 1 to 30 for use in treating or preventing a disease or condition, said implant comprising an effective amount of a bioactive agent to treat said disease or condition.

32. The implantable device for use in treating a disease or condition according to claim 31, wherein the disease or condition is selected from neoplasia, bacterial infection, microbial infection and viral infection.

33. The implantable device for use in treating a disease or condition according to claim 32, wherein the disease or condition is neoplasia.

34. The implantable device for use in treating a disease or condition according to claim 33, wherein the disease or condition is selected from lung cancer, ovarian cancer, esophageal cancer, breast cancer, colorectal cancer, gastrointestinal cancer, hepatic cancer, pancreatic cancer, head and neck cancer, skin cancer, lymphoma, sarcoma, thymoma, mesothelioma, lymphatic metastases, prostate cancer, filariasis, brucellosis, tuberculosis and HIV infection.

35. The implantable device for use in treating a disease or condition according to claim 34, wherein the disease or conditions is selected from lung cancer and lymphatic metastases of lung cancer.

36. The implantable device of any one of claims 1 to 30, wherein the bioactive agent is for the treatment or prevention of neoplasia.

37. The implantable device according to claim 36, wherein the neoplasia is cancer.

**38.** The implantable device according to claim 37, wherein the cancer is selected from lung cancer, ovarian cancer, esophageal cancer, breast cancer, colorectal cancer, gastrointestinal cancer, hepatic cancer, pancreatic cancer, head and neck cancer, skin cancer, lymphoma, sarcoma, thymoma, mesothelioma and prostate cancer.

**39.** The implantable device of any one of claims 1 to 30, wherein the bioactive agent is for treatment or prevention of metastasis to the lymphatic system.

**40.** The implantable device of any one of claims 1 to 30 for use in a method of imaging or visualizing the lymphatic system using gamma scintigraphy, Positron Emission Tomography (PET), Single Photon Emission Computer Tomography (SPECT), Magnetic Resonance Imaging (MRI), X-ray, Computer Assisted X-ray Tomography (CT), near infrared spectroscopy or ultrasound, comprising administering an implantable device according to claim 1 to a subject and performing gamma scintigraphy, Positron Emission Tomography (PET), Single Photon Emission Computer Tomography (SPECT), Magnetic Resonance Imaging (MRI), X-ray, Computer Assisted X-ray Tomography (CT), near infrared spectroscopy, or ultrasound to image or visualize the lymphatic system, wherein the implantable device comprises bioactive agents that are suitable contrast or imaging agents.

**41.** The implantable device according to claim 40, wherein the contrast or imaging agent is selected from ferromagnetic materials, perfluorochemicals, dyes, gamma emitting radiolabels and positron emitting radiolabels.

**42.** A process of preparing an implantable device according to any one of claims 1 to 30 comprising:

> a. combining the bioactive agent and the particle forming material in a suitable solvent to form a solution or suspension;
> b. spray drying the solution or suspension to form the micro or nanoparticles of the bioactive complex;
> c. combining the particles formed in b) with a biocompatible polymer suitable for forming the biocompatible and biodegradable cross-linked matrix in suitable solvents;
> d. removing the solvent of c) to form the implantable device.

**Patentansprüche**

**1.** Eine implantierbare Vorrichtung, die eine biokompatible und biologisch abbaubare vernetzte Matrix ist, die mit einem bioaktiven Komplex imprägniert ist, der für das selektive Targeting des lymphatischen Systems geeignet ist, wobei

der bioaktive Komplex in Form von Mikro- oder Nanopartikeln oder einer Kombination von Mikro- oder Nanopartikeln vorliegt, die eine Teilchengröße von 10 nm bis 11,2 $\mu$m haben, die ein oder mehrere Partikel bildende Materialien und ein oder mehrere bioaktive Mittel enthalten und die zur Abgabe der bioaktiven Mittel an das lymphatische System geeignet sind, die von einer ausreichenden Größe sind, in das lymphatische System einzudringen und dieses selektiv anzusteuern und die dazu in der Lage sind, durch die Lymphgefäße zu wandern oder in den Lymphknoten zurückgehalten zu werden;

die biokompatible und biologisch abbaubare vernetzte Matrix derart ausgewählt ist, dass der Abbau der Matrix für die Freisetzung des bioaktiven Komplexes über einen ersten Zeitraum sorgt; und

die Mikro- oder Nanopartikel des bioaktiven Komplexes so hergestellt sind, dass das bioaktive Mittel innerhalb des lymphatischen Systems über einen zweiten Zeitraum freigesetzt wird.

**2.** Die implantierbare Vorrichtung nach Anspruch 1, die für die Implantation in die Pleuralhöhle, die Bauchhöhle, ein subkutanes Kompartiment oder vaginale oder rektale Implantation geeignet ist.

**3.** Die implantierbare Vorrichtung nach Anspruch 1 oder Anspruch 2, wobei die Partikelgröße von 10 nm bis 50 nm, von 50 nm bis 100 nm, von 140 nm bis 1500 nm, von 1 $\mu$m bis 5 $\mu$m oder von 0,3 $\mu$m bis 11,2 $\mu$m ist.

**4.** Die implantierbare Vorrichtung nach Anspruch 1 oder Anspruch 2, wobei die Partikelgröße von 50 nm bis 11,2 $\mu$m ist.

**5.** Die implantierbare Vorrichtung nach Anspruch 1 oder Anspruch 2, wobei die Partikelgröße von 0,7 $\mu$m bis 2 $\mu$m ist.

**6.** Die implantierbare Vorrichtung nach einem der Ansprüche 1 bis 5, wobei das eine oder die mehreren bioaktiven Mittel ein therapeutisches Mittel ist bzw. sind.

**7.** Die implantierbare Vorrichtung nach einem der Ansprüche 1 bis 6, wobei das eine oder die mehreren bioaktiven Mittel ausgewählt wird bzw. werden aus der Gruppe bestehend aus Radioisotopen, Photosensibilisatoren, Radiosensibilisatoren, Radioprotektoren, photodynamischen Mitteln, Neutroneneinfangmitteln, Antigenen, Vakzinen, DNA RNA, Peptiden, Biomodulatoren (biological response modifiers), antimikrobiellen Mitteln und antiproliferativen Mitteln.

**8.** Die implantierbare Vorrichtung nach einem der Ansprüche 1 bis 7, wobei das eine oder die mehreren biologische(n) Mittel ausgewählt wird bzw. werden aus der Gruppe bestehend aus antiproliferativen Mitteln und antimetastatischen Mitteln.

**9.** Die implantierbare Vorrichtung nach Anspruch 8, wobei die antiproliferativen Mittel und die antimetastatischen Mittel ausgewählt werden aus der Gruppe bestehend aus Mikrotubuli Stabilisatoren, Alkylierungsmitteln, Antimetaboliten, Epidophyllotoxin, antineoplastischen Enzymen, Topoisomerase Inhibitoren, Procarbazin, Mitoxantron, Platinkoordinationskomplexen, Biomodulatoren und Wachstumsinhibitoren und blutbildenden Wachstumsfaktoren.

**10.** Die implantierbare Vorrichtung nach Anspruch 9, wobei das antineoplastische Mittel ausgewählt wird aus einem Anthracyclin Arzneimittel, Vinca Arzneimittel, Mitomycin Arzneimittel, Bleomycin Arzneimittel, cytotoxischen Nukleosiden, Taxolen, Epothilonen, Discodermolid, Pteridin Arzneimitteln, Diynenen und Podophyllotoxinen.

**11.** Die implantierbare Vorrichtung nach Anspruch 9 wobei das bioaktive Mittel ausgewählt wird aus Doxorubicin, Carminomycin, Daunorubicin, Aminopterin, Methotrexat, Methopterin, Dichlormethotrexat, Mitomycin C, Porfiromycin, Trastuzumab, 5-Fluorouracil, 6-Mercaptopurin, Gemcitabin, Cytosin-Arabinosid, Podophyllotoxin, Etoposid, Etoposidphosphat, Teniposid, Melphalan, Vinblastin, Vincristin, Leurosidin, Vindesin, Leurosin, Paclitaxel, Estramustin, Cisplatin, Carboplatin, Cyclophosphamid, Bleomycin, Gemcitibin, Ifosamid, Melphalan, Hexamethylmelamin, Thiotepa, Cytarabin, Idatrexat, Trimetrexat, Dacarbazin, L-Asparaginase, Camptothecin, CPT-11, Topotecan, Pyridobenzoindolen, Interferonen und Interleukinen.

**12.** Die implantierbare Vorrichtung nach Anspruch 11, wobei das bioaktive Mittel ausgewählt wird aus Paclitaxel und Doxorubicin.

**13.** Die implantierbare Vorrichtung nach einem der Ansprüche 1 bis 7 wobei das bioaktive Mittel ausgewählt wird aus Losten, Alkylsulfonaten, Nitrosoharnstoffen, Triazenen, Antimetaboliten, Pyrimidinen, Purinen, Naturstoffen, Epipodophyllotoxinen, Antibiotika, Enzymen, substituierten Harnstoffen, Methylhydrazinen, Adrenokortikosuppressoren, Hormonen und Antagonisten, Adrenokortikosteroiden, Progestinen, Östrogenen, Antiöstrogenen und Androgenen.

**14.** Die implantierbare Vorrichtung nach Anspruch 13, wobei das bioaktive Mittel ausgewählt wird aus Mechlorethamin, Cyclophosphamid, Melphatan, Chlorambucil, Busulfan, Carmustin, Lomustin, Semustin, Streptozocin, Dacarbazin, Folsäure, Methotrexat, Fluoruracil Cytarabin, Mercaptopurin, Thioguanin, Vinca Alkaloiden, Vinblastin, Vincristin, Vendesin, Etoposid, Teniposid, Dactinomycin, Daunorubicin, Doxorubicin, Bleomycin, Plicamycin, Mitomycin, L-Asparaginase, Hydroxyharnstoff, Procarbazin, Mitotan, Aminoglutethimid, Adrenokortikosteroiden, Prednison, Hydroxyprogesteroncaproat, Methoxyprogesteronacetat, Megestrolacetat, Diethylstilboestrol, Ethinyloestradiol, Tamoxifen, Testosteronpropionat und Fluoxymesteron.

**15.** Die implantierbare Vorrichtung nach einem der Ansprüche 1 bis 7, wobei das bioaktive Mittel ausgewählt wird aus einem Lymphangiogenesesuppressor, einem Angiogenesesuppressor, einem cytostatischen Mittel, Makromolekülen, Antioxidantien, Cytokinen, Chemokinen, Antisense Oligonucleotiden, LyP-1 Peptide-beschichteten Qdots zum Einfinden in Lymphgefäße, Hormonen und Hormonantagonisten.

**16.** Die implantierbare Vorrichtung nach Anspruch 15, wobei das bioaktive Mittel ausgewählt wird aus Vascular Endothelial Growth Factor C, D (VEGF-C,D) Antikörper, dem Antikörper zum VEGF-C,D Rezeptor (VEGFR-3), und Epidermal Growth Factor Receptor (EGFR) Antikörper.

**17.** Die implantierbare Vorrichtung nach einem der Ansprüche 1 bis 16, wobei das partikelbildende Material ausgewählt wird aus der Gruppe bestehend aus biokompatiblen Polymeren, Lipiden, Liposomen, metallischen Partikeln, magnetischen Partikeln, Biotin, Avidin und Polysacchariden.

**18.** Die implantierbare Vorrichtung nach Anspruch 17, wobei das partikelbildende Material ausgewählt wird aus der Gruppe bestehend aus Polylactid, Polyglycolsäure (PGA), Polylactid-co-Glycolid (PLGA), Polylactid (PLA), Polyvinylpyrrolidonen (PVP), Polylactid-co-Caprolacton, Polyethylenglykol (PEG), Polyethylenoxid (PEO), Polystyrol, Polylactid-block-Polyethylenglycol, Polyglycolsäure-block-Polyethylenglycol, Polylactid-co-Glycolid-block-Polyethylenglycol, Polyethylenglycol-block-Lipid, Polyvinylalkohol (PVA), Polyester, Polyorthoester, Polyphosphazin, Polyphosphatester, Polycaprolactaonen, Gelatin, Collagen, einem Glycosaminoglycan, Polyorthoestern, Polysacchariden, Polyhyaluronsäure, Polyalginsäure, Chitin, Chitosan, Cellulose, Hydroxyethylcellulose, Hydroxypropylcellulose, Carboxymethylcellulose, Polypeptiden, Polylysinen, Polyglutaminsäure, Albumin, Polyanhydriden, Polyhydroxyalkanoaten, Polyhydroxyvalerat, Polyhydroxybutyrat, Proteinen, Polyphosphatestern, Polyacrylamid (PAA), und Mischungen davon.

**19.** Die implantierbare Vorrichtung nach Anspruch 18, wobei das partikelbildende Material PLGA ist.

**20.** Die implantierbare Vorrichtung nach einem der Ansprüche 1 bis 19, wobei der bioaktive Komplex Additive umfasst.

**21.** Die implantierbare Vorrichtung nach Anspruch 20, wobei die Additive ausgewählt werden aus Zusatzstoffen, Überzugsmitteln, Farbstoffen, Bindemitteln, Puffern, Schmierstoffen, Sprengmitteln, Weichmachern und Stabilisatoren.

**22.** Die implantierbare Vorrichtung nach einem der Ansprüche 1 bis 21, wobei der bioaktive Komplex Mikropartikel von PLGA und Paclitaxel oder Lipid und Doxorubicin umfasst.

**23.** Die implantierbare Vorrichtung nach einem der Ansprüche 1 bis 22, wobei sich die Matrix über einen Zeitraum von mehreren Stunden bis etwa einem Jahr abbaut, insbesondere von mehreren Tagen bis mehreren Wochen.

**24.** Die implantierbare Vorrichtung nach einem der Ansprüche 1 bis 23, wobei die Matrix formbar ist.

**25.** Die implantierbare Vorrichtung nach einem der Ansprüche 1 bis 24, wobei die Matrix in einer Form vorliegt, die ausgewählt wird aus der Gruppe bestehend aus einem Schwamm, einer Folie, einem Film, einem Netz, einem Bausch, einem Tampon und einem Polster.

**26.** Die implantierbare Vorrichtung nach einem der Ansprüche 1 bis 25, wobei die biokompatible Matrix ein Hydrogelfilm ist.

**27.** Die implantierbare Vorrichtung nach einem der Ansprüche 1 bis 25, wobei die Matrix ausgewählt wird aus der Gruppe bestehend aus Gelatine, Collagen, Gelatine-Alginat.

**28.** Die implantierbare Vorrichtung nach einem der Ansprüche 1 bis 27, wobei die Matrix Additive enthält.

**29.** Die implantierbare Vorrichtung nach Anspruch 28, wobei das Additiv ein radioopakes Material ist, dass durch Röntgenstrahlen nachweisbar ist.

**30.** Die implantierbare Vorrichtung nach einem der Ansprüche 1 bis 29, wobei ein oder mehrere freie bioaktive Mittel in die biokompatible und bioaktive Matrix eingebaut sind.

**31.** Die implantierbare Vorrichtung, wie in einem der Ansprüche 1 bis 30 definiert, zur Verwendung bei der Behandlung oder Vorbeugung einer Krankheit oder eines Zustands, wobei das Implantat eine effektive Menge eines bioaktiven Mittels umfasst, um die Krankheit oder den Zustand zu behandeln.

**32.** Die implantierbare Vorrichtung zur Verwendung bei der Behandlung einer Krankheit oder eines Zustands nach Anspruch 31, wobei die Krankheit oder der Zustand ausgewählt werden aus Neoplasie, einer bakteriellen Infektion, einer mikrobiellen Infektion und einer viralen Infektion.

**33.** Die implantierbare Vorrichtung zur Verwendung bei der Behandlung einer Krankheit oder eines Zustands nach Anspruch 32, wobei die Krankheit oder der Zustand Neoplasie ist.

**34.** Die implantierbare Vorrichtung zur Verwendung bei der Behandlung einer Krankheit oder eines Zustands nach Anspruch 33, wobei die Krankheit oder der Zustand ausgewählt werden aus Lungenkrebs, Eierstockkrebs, Speiseröhrenkrebs, Brustkrebs, Dickdarmkrebs, Magen- und Darmkrebs, Leberkrebs, Bauchspeicheldrüsenkrebs, Kopf- und Halskrebs, Hautkrebs, Lymphom, Sarkom, Thymom, Mesotheliom, lymphatischen Metastasen, Prostatakrebs, Filariasis, Brucellose, Tuberkulose und einer HIV Infektion.

**35.** Die implantierbare Vorrichtung zur Verwendung bei der Behandlung einer Krankheit oder eines Zustands nach Anspruch 34, wobei die Krankheit oder der Zustand ausgewählt werden aus Lungenkrebs und lymphatischen Metastasen von Lungenkrebs.

**36.** Die implantierbare Vorrichtung nach einem der Ansprüche 1 bis 30, wobei das bioaktive Mittel zur Behandlung oder Vorbeugung von Neoplasie ist.

**37.** Die implantierbare Vorrichtung nach Anspruch 36, wobei die Neoplasie Krebs ist.

**38.** Die implantierbare Vorrichtung nach Anspruch 37, wobei der Krebs ausgewählt wird aus Lungenkrebs, Eierstockkrebs, Speiseröhrenkrebs, Brustkrebs, Dickdarmkrebs, Magen- und Darmkrebs, Leberkrebs, Bauchspeicheldrüsenkrebs, Kopf- und Halskrebs, Hautkrebs, Lymphom, Sarkom, Thymom, Mesotheliom und Prostatakrebs.

**39.** Die implantierbare Vorrichtung nach einem der Ansprüche 1 bis 30, wobei das bioaktive Mittel zur Behandlung oder Vorbeugung von Metastasen am lymphatischen System ist.

**40.** Die implantierbare Vorrichtung nach einem der Ansprüche 1 bis 30, zur Verwendung bei einem Verfahren zur Abbildung oder Sichtbarmachung des lymphatischen Systems unter Verwendung von Gamma Szintigraphie, Positronen Emissions Tomographie (PET), Single Photon Emissions Computer Tomographie (SPECT), Magnetresonanztomographie (MRI), Röntgen, Computer Tomographie (CT), Nahinfrarotspektroskopie oder Ultraschall, welches das Verabreichen einer implantierbaren Vorrichtung nach Anspruch 1 an einen Patienten und das Durchführen von Gamma Szintigraphie, Positronen Emissions Tomographie (PET), Single Photon Emissions Computer Tomographie (SPECT), Magnetresonanztomographie (MRI), Röntgen, Computer Tomographie (CT), Nahinfrarotspektroskopie oder Ultraschall umfasst, um das lymphatische System abzubilden oder sichtbar zu machen, wobei die implantierbare Vorrichtung bioaktive Mittel umfasst, die geeignete Kontrast- oder Bildgebungsmittel sind.

**41.** Die implantierbare Vorrichtung nach Anspruch 40, wobei das Kontrast- oder Bildgebungsmittel ausgewählt wird aus Ferromagnetika, Perfluorchemikalien, Farben, gammastrahlenden Radiomarkern und positronenemittierenden Radiomarkern.

**42.** Verfahren zur Herstellung einer implantierbaren Vorrichtung nach einem der Ansprüche 1 bis 30 umfassend:

 a. Kombinieren des bioaktiven Mittels und des Partikel bildenden Materials in einem geeigneten Lösungsmittel, um eine Lösung oder eine Suspension zu bilden;
 b. Sprühtrocknen der Lösung oder der Suspension, um die Mikro- oder Nanopartikel des bioaktiven Komplexes zu bilden;
 c. Kombinieren der Partikel, die in b) gebildet wurden, mit einem biokompatiblen Polymer, dass zur Bildung der biokompatiblen und biologisch abbaubaren vernetzten Matrix geeignet ist, in geeigneten Lösungsmitteln;
 d. Entfernen des Lösungsmittels von c), um die implantierbare Vorrichtung zu bilden.

## Revendications

**1.** Un dispositif implantable qui consiste en une matrice réticulée biocompatible et biodégradable imprégnée d'un complexe bioactif approprié pour cibler sélectivement le système lymphatique,
dans lequel

 - le complexe bioactif est sous forme de microparticules, de nanoparticules ou de mélanges de microparticules et de nanoparticules présentant une taille de particules de 10 nm à 11,2 $\mu$m qui comprennent un ou plusieurs matériaux générateurs de particules et un ou plusieurs agents bioactifs et qui conviennent pour délivrer des agents bioactifs au système lymphatique, présentant une dimension suffisante pour pénétrer dans le système lymphatique et sélectivement le cibler et étant capables de migrer dans les vaisseaux lymphatiques ou d'être retenus dans les nodules lymphatiques;
 - la matrice réticulée biocompatible et biodégradable est choisie telle que la dégradation de la matrice fournisse une libération du complexe bioactif sur une première période de temps; et
 - les microparticules ou nanoparticules du complexe bioactif sont préparés de façon que l'agent bioactif soit libéré dans le système lymphatique pendant une seconde période de temps.

**2.** Le dispositif implantable selon la revendication 1, qui convient pour une implantation dans la cavité pleurale, dans la cavité péritonéale, dans un compartiment sous-cutanée, par voie vaginale ou voie rectale.

**3.** Le dispositif implantable selon la revendication 1 ou la revendication 2, dans lequel la taille des particules est de 10 nm à 50 nm, de 50 nm à 100 nm, de 140 nm à 1500 nm, de 1 $\mu$m à 5 $\mu$m ou de 0,3 $\mu$m à 11,2 $\mu$m.

**4.** Le dispositif implantable selon la revendication 1 ou la revendication 2, dans lequel la taille des particules est de 50 nm à 11,2 $\mu$m.

**5.** Le dispositif implantable selon la revendication 1 ou la revendication 2, dans lequel la taille des particules est de 0,7 $\mu$m à 2 $\mu$m.

**6.** Le dispositif implantable selon l'une quelconque des revendications 1 à 5, dans lequel un ou plusieurs des agents bioactifs sont des agents thérapeutiques.

**7.** Le dispositif implantable selon l'une quelconque des revendications 1 à 6, dans lequel un ou plusieurs des agents bioactifs sont choisis dans le groupe consistant en des radio-isotopes, des photosensibilisateurs, des radiosensibilisateurs, des agents radioprotecteurs, des agents photodynamiques, des agents de capture de neutrons, des antigènes, des vaccins, ADN, ARN, des peptides, des modificateurs de la réponse biologique, des agents antimicrobiens et des agents anti-prolifératifs.

**8.** Le dispositif implantable selon l'une quelconque des revendications 1 à 7, dans lequel un ou plusieurs des agents bioactifs sont choisis dans le groupe constitué par les agents anti-prolifératifs et les agents anti-métastasiques.

**9.** Le dispositif implantable selon la revendication 8, dans lequel les agents antiprolifératifs et les agents anti-métastasiques sont choisis dans le groupe constitué par les agents de stabilisation des microtubules, les agents alkylants, les antimétabolites, les épidophyllotoxines, les enzymes antinéoplasiques, les inhibiteurs de topoisomérase, la procarbazine, la mitoxantrone, les complexes de coordination du platine, les modificateurs de la réponse biologique, les inhibiteurs de croissance et les facteurs de croissance hématopoïétiques.

**10.** Le dispositif implantable selon la revendication 9, dans lequel l'agent antinéoplasique est choisi dans le groupe comprenant: les médicaments à base d'anthracycline, les médicaments à base de vinca, les médicaments à base de mitomycine, les médicaments à base de bléomycine, les nucléosides cytotoxiques, les taxanes, les épothilones, le discodermolide, les médicaments à base de ptéridine, les diynènes et les podophyllotoxines.

**11.** Le dispositif implantable selon la revendication 9, dans lequel l'agent bioactif est choisi dans le groupe comprenant: la doxorubicine, la carminomycine, la daunorubicine, l'aminoptérine, le méthotrexate, la méthoptérine, le dichlorométhotrexate, la mitomycine C, la porfiromycine, le trastuzumab, le 5-fluorouracile, la 6-mercaptopurine, la gemcitabine, le cytosine arabinoside, la podophyllotoxine, l'étoposide, le phosphate d'étoposide, le téniposide, le melphalan, la vinblastine, la vincristine, la leurosidine, la vindésine, la leurosine, le paclitaxel, l'estramustine, le cisplatine, le carboplatine, le cyclophosphamide, la bléomycine, gemcitibine, l'ifosamide, le melphalan, l'hexaméthyl mélamine, le thiotépa, la cytarabine, l'idatrexate, le trimétrexate, la dacarbazine, la L-asparaginase, la camptothécine, le CPT-11, le topotécan, les dérivés pyridobenzoindole, les interférons et les interleukines.

**12.** Le dispositif implantable selon la revendication 11, dans lequel l'agent bioactif est choisi dans le groupe comprenant le paclitaxel et la doxorubicine.

**13.** Le dispositif implantable selon l'une quelconque des revendications 1 à 7, dans lequel l'agent bioactif est choisi dans le groupe comprenant: les moutardes azotées, les alkylsulfonates, les nitrosourées, les triazènes, les antimétabolites, les pyrimidines, les purines, les produits naturels, les épipodophyllotoxines, les antibiotiques, les enzymes, les urées substituées, les dérivés de méthylhydrazine, les hormones, les antagonistes et les suppresseurs de l'adrénocorticoïde, les adrénocorticostéroïdes, les progestatifs, les oestrogènes, les anti-oestrogènes et les androgènes.

**14.** Le dispositif implantable selon la revendication 13, dans lequel l'agent bioactif est choisi dans le groupe comprenant la méchloréthamine, le cyclophosphamide, le melphatan, le chlorambucil, le busulfan, la carmustine, la lomusine, la sémustine, la streptozocine, la dacarbazine, l'acide folique, le méthotrexate, le fluorouracil cytarabine, la mercaptopurine, la thioguanine, des alcaloïdes de la vinca, la vinblastine, la vincristine, vendesine, l'étoposide, le téniposide, la dactinomycine, la daunorubicine, la doxorubicine, la bléomycine, la plicamycine, la mitomycine, la L-asparaginase, l'hydroxyurée, la procarbazine, le mitotane, l'aminoglutéthimide, les adrénocorticostéroïdes, la prednisone, le caproate d'hydroxyprogestérone, l'acétate de méthoxyprogestérone, l'acétate de mégestrol, le diéthylstilbestrol, l'éthinyloestradiol, le tamoxifène, le propionate de testostérone et la fluoxymestérone.

15. Le dispositif implantable selon l'une quelconque des revendications 1 à 7, dans lequel l'agent bioactif est choisi dans le groupe comprenant: les suppresseurs de lymphangiogénèse, les suppresseurs de l'angiogénèse, les agents cytostatiques, les macromolécules, les antioxydants, les cytokines, les chimiokines, les oligonucléotides antisens, les nanopoints revêtus de peptides LyP-1 dans les hôtes pour lymphatiques, les hormones et les antagonistes hormonaux.

16. Le dispositif implantable selon la revendication 15, dans lequel l'agent bioactif est choisi dans le groupe des facteurs de croissance C endothéliale vasculaires, des anticorps D (VEGF C,D), des anticorps de récepteur de VEGF-C,D (VEGFR-3), et des anticorps de récepteurs du facteur de croissance épidermique (EGFR).

17. Le dispositif implantable selon l'une quelconque des revendications 1 à 16, dans lequel le matériau générateur de particules est choisi dans le groupe constitué par les polymères biocompatibles, les lipides, les liposomes, les particules métalliques, les particules magnétiques, la biotine, l'avidine et les polysaccharides.

18. Le dispositif implantable selon la revendication 17, dans lequel le matériau générateur de particules est choisi dans le groupe constitué par l'acide polylactique, l'acide polyglycolique (PGA), l'acide polylactique-co-glycolique (PLGA), l'acide poly-lactique (PLA), les polyvinylpyrrolidones (PVP), l'acide polylactique-co-caprolactone, le polyéthylene glycol (PEG), l'oxyde de polyéthylène (PEO), le polystyrène, l'acide polylactique polyéthylèneglycol-bloc, l'acide poly glycolique polyéthylèneglycol-bloc, le poly lactide-co-glycolide-bloc-polyéthylène glycol, le polyéthylène glycol-bloc-lipide, l'alcool polyvinylique (PVA), le polyester, les poly-orthoesters, la poly(phosphazine), les poly(ester phoasphate), les polycaprolactaones, la gélatine, le collagène, les glycosaminoglycanes, les polyorthoesters, les polysaccharides, l'acide polyhyaluronique, l'acide polyalginique, la chitine, le chitosane, la cellulose, l'hydroxyéthylcellulose, l'hydroxypropylcellulose, la carboxyméthylcellulose, les polypeptides, la polylysine, l'acide polyglutamique, l'albumine, les polyanhydrides, les polyhydroxyalkonoates, le polyhydroxy valérate, le polyhydroxybutyrate, les protéines, les polyphosphates esters, le polyacrylamide (PAA) et leurs mélanges.

19. Le dispositif implantable selon la revendication 18, dans lequel le matériau générateur de particules est PLGA.

20. Le dispositif implantable selon l'une quelconque des revendications 1 à 19, dans lequel le complexe bioactif comprend des additifs.

21. Le dispositif implantable selon la revendication 20, dans lequel les additifs sont choisis parmi des adjuvants, des revêtements, des colorants, des liants, des tampons, des lubrifiants, des désintégrants, des plastifiants et des stabilisants.

22. Le dispositif implantable selon l'une quelconque des revendications 1 à 21, dans lequel le complexe bioactif comprend des microparticules de PLGA et de paclitaxel ou de doxorubicine et d'un lipide.

23. Le dispositif implantable selon l'une quelconque des revendications 1 à 22, dans lequel la matrice se dégrade sur une période de temps de plusieurs heures à environ 1 an, notamment de plusieurs jours à plusieurs semaines.

24. Le dispositif implantable selon l'une quelconque des revendications 1 à 23, dans lequel la matrice est façonnable.

25. Le dispositif implantable selon l'une quelconque des revendications 1 à 24, dans lequel la matrice est sous une forme choisie dans le groupe consistant en éponges, feuilles, films, mailles, compresses, tampons et patch.

26. Le dispositif implantable selon l'une quelconque des revendications 1 à 25, dans lequel la matrice biocompatible est un film d'hydrogel.

27. Le dispositif implantable selon l'une quelconque des revendications 1 à 25, dans lequel la matrice est choisie dans le groupe constitué par la gélatine, le collagène, la gélatine-alginate.

28. Le dispositif implantable selon l'une quelconque des revendications 1 à 27, dans lequel la matrice contient des additifs.

**29.** Le dispositif implantable selon la revendication 28, dans lequel l'additif est un matériau radio-opaque détectable par rayons X.

**30.** Le dispositif implantable selon l'une quelconque des revendications 1 à 29, dans lequel un ou plusieurs agents bioactifs libres sont incorporés dans la matrice biocompatible et bioactive.

**31.** Un dispositif implantable tel que défini dans l'une quelconque des revendications 1 à 30, pour être utilisé pour le traitement ou la prévention d'une maladie ou d'une affection, ledit implant comprenant une quantité efficace d'un agent bioactif pour traiter ladite maladie ou affection.

**32.** Le dispositif implantable pour être utilisé pour le traitement ou la prévention d'une maladie ou d'une affection selon la revendication 31, dans lequel la maladie ou l'affection est choisi dans le groupe comprenant: néoplasie, infection bactérienne, infection microbienne et infection virale.

**33.** Le dispositif implantable pour être utilisé pour le traitement ou la prévention d'une maladie ou d'une affection selon la revendication 32, dans lequel la maladie ou l'affection est une néoplasie.

**34.** Le dispositif implantable pour être utilisé pour le traitement ou la prévention d'une maladie ou d'une affection selon la revendication 33, dans lequel la maladie ou l'affection est choisie parmi le cancer du poumon, le cancer de l'ovaire, le cancer de l'oesophage, le cancer du sein, le cancer colorectal, le cancer gastro-intestinal, le cancer du foie, le cancer du pancréas, le cancer tête et cou, le cancer de la peau, les lymphomes, les sarcomes, les thymomes, les mésothéliomes, les métastases lymphatiques, le cancer de la prostate, la filariose, la brucellose, la tuberculose et l'infection VIH.

**35.** Le dispositif implantable pour être utilisé pour le traitement ou la prévention d'une maladie ou d'une affection selon la revendication 34, dans lequel la maladie ou l'affection est choisie parmi le cancer du poumon et les métastases lymphatiques du cancer du poumon.

**36.** Le dispositif implantable selon une quelconque des revendications 1 à 30, dans lequel l'agent bioactif est pour le traitement ou la prévention des néoplasies.

**37.** Le dispositif implantable selon la revendication 36, dans lequel le néoplasie est le cancer.

**38.** Le dispositif implantable selon la revendication 37, dans lequel le cancer est choisi parmi le cancer du poumon, le cancer de l'ovaire, cancer de l'oesophage, le cancer du sein, le cancer colorectal, le cancer gastro-intestinal, le cancer du foie, le cancer du pancréas, le cancer tête et cou, le cancer de la peau, les lymphomes, les sarcomes, les thymomes, les mésothéliomes et le cancer de la prostate.

**39.** Le dispositif implantable selon l'une quelconque des revendications 1 à 30, dans lequel l'agent bioactif est destiné au traitement ou à la prévention des métastases dans le système lymphatique.

**40.** Le dispositif implantable selon l'une quelconque des revendications 1 à 30, pour être utilisé dans un procédé d'imagerie ou de visualisation du système lymphatique faisant emploi d'une scintigraphie gamma, tomographie par émission de positrons (PET), tomographie par émission informatique à photon unique (SPECT), imagerie par résonance magnétique (IRM), rayons X, tomographie par rayons X assistée par ordinateur (CT), spectroscopie à proche infrarouge ou à ultrasons, comprenant l'administration d'un dispositif implantable selon la revendication 1 à un sujet et la mise en oeuvre de la scintigraphie gamma, la tomographie par émission de positrons (PET), tomographie par émission informatique à photon unique (SPECT), imagerie par résonance magnétique (IRM), rayons-X, tomographie par rayons X assistée par ordinateur (CT), spectroscopie à proche infrarouge ou à ultrasons pour représenter l'image ou visualiser le système lymphatique, dans lequel le dispositif implantable comprend des agents bioactifs qui sont des agents de contraste ou d'imagerie appropriées.

**41.** Le dispositif implantable selon la revendication 40, dans lequel l'agent de contraste ou d'imagerie est choisi dans le groupe comprenant: les matériaux ferromagnétiques, les produits chimiques perfluorés, les colorants, les marqueurs radioactifs émettant des rayons gamma et les marqueurs radioactifs émettant des positrons.

**42.** Un procédé de préparation d'un dispositif implantable selon l'une quelconque des revendications 1 à 30, comprenant :

a) la combinaison de l'agent actif et du matériau générateur de particules dans un solvant approprié pour former une solution ou suspension;

b) le séchage par atomisation de la solution ou suspension pour former des microparticules ou nanoparticules du complexe bioactif;

c) la combinaison des particules formées à l'étape b) avec un polymère biocompatible approprié pour former une matrice réticulée biocompatible et biodégradable dans des solvants appropriés;

d) l'élimination du solvant de l'étape c) pour former le dispositif implantable.

# Biodistribution of $^{111}$In-aminopolystyrene via intrapleural administration

FIGURE 1

EP 1 922 094 B1

FIGURE 2

# In vitro releasing profile of PLGA-PTX

FIGURE 3

FIGURE 4

FIGURE 5

FIGURE 6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20040234597 A
- US 5741331 A
- US 4946899 A
- US 6545097 B
- US 27539101 P
- US 2465357 A
- US 5741516 A

**Non-patent literature cited in the description**

- COLEMAN. *Int J Radiation Onc,* 1998, vol. 42 (4), 781-783
- BARTH et al. *Cancer Res,* 1990, vol. 50, 1061-1071
- KOHNO et al. *J Infect Chemother,* 1998, vol. 4, 159-173
- LASIC et al. Vesicles. Marcel Dekker, 1996, 477-489
- BOOKMAN. *Curr Opin Oncol,* 1995, vol. 7 (5), 478-484
- ALVING. *J Immuno Methods,* 1991, vol. 140, 1-13
- DAEMEN. Medical Applications of Liposomes. Elsevier Science B.V, 1998, 117-143
- GREGORIADIS et al. Medical Applications of Liposomes. Elsevier Science B.V, 1998, 61-73
- WOODLE et al. Medical Applications of Liposomes. Elsevier Science B.V, 1998, 429-449
- LIU J et al. *Lung Cancer,* 2006, vol. 51 (3), 377-86
- PREVO R et al. *J. Biol. Chem.,* 2001, vol. 276, 19420-19430
- WEISS, L. et al. *Int J Cancer,* 1987, vol. 40 (4), 570-4
- MOUNTAIN CF. *Chest,* 1977, vol. 111, 1718-23
- KUO TH. *Anticancer Res,* 1993, vol. 13, 293-7
- MARTINI N J. *Thorac Cardiovas Surg,* 1995, vol. 109, 120-9
- MARTINEZ SR et al. *Clin Colorectal Cancer,* 2005, vol. 4, 320-4
- MCGUIRE WL. *Breast Cancer Res.,* 1987, vol. 10, 5-9
- BERGQUIST et al. *Seminars in Nuclear Med.,* 1983, vol. 12, 9-10
- STRAND, S. E. *CRC Crit. Rev. Drug Carrier System,* 1989, vol. 6, 211-237
- KALEDIN, V. I. et al. *J Natl Cancer Inst.,* 1981, vol. 66, 881-7
- KHATO, J et al. *Cancer Treat Rep.,* 1982, vol. 66, 517-27
- KONNO, H et al. *Jpn J Surg.,* 1990, vol. 20, 424-8
- HAGIWARA, A et al. *Anticancer Drug Des.,* 1987, vol. 1, 313-21
- HAGIWARA, A et al. *Anticancer Drugs,* 1997, vol. 8, 666-70
- KARAJGI, J. S. et al. *J Microencapsul.,* 1994, vol. 11, 539-45
- YOSHIMURA, K et al. *Gan To Kagaku Ryoho.,* 1996, vol. 23, 1519-22
- PORTER, C. J et al. *J Pharm Sci.,* 1996, vol. 85, 351-6
- YOSHIKAWA, H et al. *Biol Pharm Bull.,* 1996, vol. 19, 1527-9
- NAKAMURA, Y et al. *Surg Today.,* 2002, vol. 32, 335-42
- SHINOHARA H. *Anat Rec,* 1997, vol. 249 (1), 16-23
- SHIMADA M. *Anticancer Drug Des,* 1993, vol. 8, 249-55
- HAWLEY AE ; ILLUM L ; DAVIS SS. Preparation of biodegradable, surface engineered PLGA nanospheres with enhanced lymphatic drainage and lymph node uptake. *Pharm Res.,* 1997, vol. 14, 657-61
- MAINCENT P ; THOUVENOT P ; AMICABILE C et al. Lymphatic targeting of polymeric nanoparticles after intraperitoneal administration in rats. *Pharm Res.,* 1992, vol. 9, 1534-9
- TOKUDA K ; NATSUGOE S ; SHIMADA M et al. Design and testing of a new cisplatin form using a base material by combining poly-D,L-lactic acid and polyethylene glycol acid against peritoneal metastasis. *Int J Cancer,* 1998, vol. 76, 709-12
- LIGGINS TR ; D'AMOURS S ; DEMETRICK JS et al. Paclitaxel loaded poly(L-lactic acid) microspheres for the prevention of intraperitoneal carcinomatosis after a surgical repair and tumor cell spill. *Biomaterials,* 2002, vol. 21, 1959-69

- **HAWLEY AE ; ILLUM L ; DAVIS SS.** Preparation of biodegradable, surface engineered PLGA nanospheres with enhanced lymphatic drainage and lymph node uptake. *Pharm Res,* 1997, vol. 14, 657-61
- **WONG HL et al.** *J Pharmacol Exp Ther.,* 2006, vol. 317, 1372-1381
- **WONG HL et al.** *Pharm Res.,* 2006, vol. 23, 1574-85
- **KENNEDY L ; HARLEY RA ; SAHN SA ; STRANGE C.** Talc slurry pleurodesis. Pleural fluid and histologic analysis. *Chest,* 1995, vol. 107, 1707-12
- **SINGER JJ ; J.J.T. LEAL.** Aseptic pleuritis experimentally produced. *J Thorac Surg,* 1941, vol. 10, 251-83
- **KARSNER HT SC.** The removal of particulate matter from the pleura. *J Med Res,* 1920, vol. 42, 91-8
- **MEDINA LA ; KLIPPER R ; PHILLIPS WT ; GOINS B.** Pharmacokinetics and biodistribution of [111In]-avidin and [99mTc]-biotin-liposomes injected in the pleural space for the targeting of mediastinal nodes. *Nucl Med Biol.,* 2004, vol. 31, 41-51
- **HAWLEY AE ; ILLUM L ; DAVIS SS.** Preparation of biodegradable, surface engineered PLGA nanospheres with enhanced lymphatic drainage and lymph node uptake. *Pharm Res,* 1997, vol. 14 (5), 657-61
- **CHARMAN, WILLIAM N.** Lymphatic Transport of Drugs. CRC Press, 1992, 279-315
- **BETTENDORF U.** Electronmicroscopic studies on the peritoneal resorption of intraperitoneally injected latex particles via the diaphragmatic lymphatics. *Lymphology,* 1979, vol. 12 (2), 66-70
- **HAWLEY A.E. ; DAVIS S.S. ; ILLUM L.** Targeting of colloids to lymph nodes: influence of lymphatic physiology ad colloidal characteristics. *Advanced Drug Delivery Reviews,* 1995, 129-48
- *J Control Release,* 2001, vol. 76, 239-54
- **ONSECA C.** *J Control Release.,* 2002, vol. 83, 273-286
- **LIGGINS R.** *Inflamm Res.,* 2004, vol. 53, 363-72
- **MO Y.** *J Control Release,* 28 November 2005, vol. 108, 244-62
- **GUPTE A.** *Int J Pharm.,* 2004, vol. 276, 93-106
- **MU L et al.** *J Control Release,* 2001, vol. 76, 239-54
- **HAWLEY AE et al.** *Pharm Res.,* May 1997, vol. 14 (5), 657-61
- **LIU J et al.** *Lung Cancer,* 2006, vol. 51, 377-86
- **BETTENDORF U.** *Lymphology,* 1979, vol. 12, 66-70
- **HAWLEY A.E et al.** *Pharm Res.,* 1997, vol. 14, 657-61
- **LIGGINS RT et al.** *Biomaterials,* 2000, vol. 21, 1959-69
- **FONSECA C.** *J Control Release,* 2002, vol. 83, 273-286
- **MU L et al.** *J Control Release,* 2003, vol. 86, 33-48
- **MO Y et al.** *J Control Release,* 2005, vol. 108, 244-62
- **MU, L et al.** *J Control Release,* 2001, vol. 76, 239-54
- **GILBERT DL et al.** *J Biomed Mater Res.,* 1990, vol. 24, 1221-39
- **KUIJPERS A.J et al.** *J Biomater Sci Polym Ed.,* 2000, vol. 11, 225-43
- **OLDE DAMINK LH et al.** *Biomaterials,* 1996, vol. 17, 765-73
- **PIEPER JS et al.** *Biomaterials,* 2000, vol. 21, 581-93
- **LIGGINS RT et al.** *Inflamm Res.,* August 2004, vol. 53 (8), 363-72
- **LIGGINS RT et al.** *Int J Pharm.,* 2001, vol. 222, 19-33
- **LIEBMANN J et al.** *Cancer Chemother Pharmacol.,* 1994, vol. 33, 331-9
- **LIEBMANN JE et al.** *Br J Cancer,* 1993, vol. 68, 1104-9
- **LOPES NM et al.** *Cancer Chemother Pharmacol.,* 1993, vol. 32, 235-42
- **HOWARD et al.** *Clin Exp Metastasis.,* 1999, vol. 17, 157-62
- **YAMORI T et al.** *Jpn. J. Cancer Res.,* 1997, vol. 88, 1205-10
- *J. Pharmacol. Exp. Ther.,* 1987, vol. 242, 692-698
- **HENNEQUIN C.** *Cancer Radiother.,* 2004, vol. 8 (1), 95-105
- *J Pharmacol Exp Ther.,* 2006, vol. 317, 1372-1381
- **LIU Z et al.** *J Control Rel.,* 2001, vol. 77, 213-224
- **WONG H et al.** *J Pharmacol Exp Ther.,* 2006, vol. 317, 1372-1381
- **LIU J et al.** *94th AACR,* 2003
- *Flatmark K European J Cancer,* 2004, vol. 40 (10), 1593-98
- **HOWARD RB.** *Clin Exp Metastasis,* 1999, vol. 17, 157-62
- *European J Cancer,* 2004, vol. 40 (10), 1593-98
- **BIN LU et al.** *European J Pharm Sci,* 2006, vol. 28, 86-95